# EUROPEAN PATENT SPECIFICATION

(11) **EP 2 498 773 B1**
(45) Date of publication and mention of the grant of the patent: **29.07.2015**
(21) Application number: 10807721.5
(22) Date of filing: 10.11.2010
(51) Int. Cl.: A61K 31/4184, A61K 31/454, A61K 31/4545, A61K 31/519, A61K 31/573, A61K 31/704, A61K 31/7048, A61K 45/06, A61K 38/50, A61P 35/02, G01N 33/50, G01N 33/68, G01N 33/574, C07K 16/18

(54) **COMPOSITIONS FOR THE TREATMENT OF CHEMORESISTANT LEUKAEMIA AND/OR OF POTENTIALLY CHEMORESISTANT LEUKAEMIA**
ZUSAMMENSETZUNGEN ZUR BEHANDLUNG VON CHEMORESISTENTER LEUKÄMIE UND/ODER VON POTENZIELL CHEMORESISTENTER LEUKÄMIE
NOUVELLES COMPOSITIONS POUR LE TRAITEMENT DE LEUCÉMIES CHIMIORÉSISTANTES ET/OU POTENTIELLEMENT CHIMIORÉSISTANTES

(30) Priority: 10.11.2009 IT RM20090578; 09.02.2010 IT RM20100048
(43) Date of publication of application: 19.09.2012
(73) Proprietor: Noi Per Voi Onlus, 50139 Careggi (FI) (IT)
(72) Inventor: ARCANGELI, Annarosa, I-50134 Firenze (IT); BECCHETTI, Andrea, I-20126 Milano (IT); PILLOZZI, Serena, I-50134 Firenze (IT); MASSELLI, Marika, I-50134 Firenze (IT); DE LORENZO, Emanuele, I-50134 Firenze (IT)
(74) Representative: Predazzi, Valentina
(86) International application number: PCT/IB2010/055112
(87) International publication number: WO 2011/058508

(56) References cited:
- WO-A1-2010/121141
- WO-A2-01/47507
- WO-A2-2004/006842
- WO-A2-2009/070331
- CH-A5- 681 780
- DATABASE BIOSIS [Online] BIOSCIENCES INFORMATION SERVICE, PHILADELPHIA, PA, US; November 2008 (2008-11), PILLOZZI SERENA ET AL: "A Macromolecular Signaling Complex Formed by CXCR4, VLA4 and hERG1 K+ Channels Mediates Bone Marrow-Induced Chemo-Resistance in Childhood Acute Lymphoblastic Leukemias: Shortcoming Effects of hERG1 Channels Inhibitors", XP002587813, Database accession no. PREV200900258328 & BLOOD, vol. 112, no. 11, November 2008 (2008-11), pages 575-576, 50TH ANNUAL MEETING OF THE AMERICAN- SOCIETY-OF-HEMATOLOGY; SAN FRANCISCO, CA, USA; DECEMBER 06 -09, 2008 ISSN: 0006-4971
- DATABASE BIOSIS [Online] BIOSCIENCES INFORMATION SERVICE, PHILADELPHIA, PA, US; November 2007 (2007-11), PILLOZZI SERENA ET AL: "Expression and role of hERG1 channels in pediatric acute lymphoblastic Leukemias: Shortcoming of drug resistance by hERG1 channel inhibitors in stoma-supported leukaemia cell cultures in vitro.", XP002587814, Database accession no. PREV200800215997 & BLOOD, vol. 110, no. 11, Part 1, November 2007 (2007-11), page 222A, 49TH ANNUAL MEETING OF THE AMERICAN-SOCIETY-OF-HEMATOLOGY; ATLANTA, GA, USA; DECEMBER 08 -11, 2007 ISSN: 0006-4971
- ARCANGELI A ET AL: "Targeting ion channels in cancer: A novel frontier in antineoplastic therapy", CURRENT MEDICINAL CHEMISTRY 2009 NL LNKD- DOI:10.2174/092986709787002835, vol. 16, no. 1, 2009, pages 66-93, XP002587815, ISSN: 0929-8673 cited in the application
- WANG LI ET AL: "Reversal of anticancer drug resistance by macrolide antibiotics in vitro and in vivo", CLINICAL AND EXPERIMENTAL PHARMACOLOGY AND PHYSIOLOGY, vol. 27, no. 8, August 2000 (2000-08), pages 587-593, XP002587816, ISSN: 0305-1870
- PAJEVA I K ET AL: "Membrane interactions of some catamphiphilic drugs and relation to their multidrug-resistance-reversing ability", JOURNAL OF CANCER RESEARCH AND CLINICAL ONCOLOGY, SPRINGER INTERNATIONAL, BERLIN, DE, vol. 122, no. 1, 1 January 1996 (1996-01-01), pages 27-40, XP008123342, ISSN: 0171-5216
- SOLARY E ET AL: "SUFFICIENT LEVELS OF QUININE IN THE SERUM CIRCUMVENT THE MULTIDRUG RESISTANCE OF THE HUMAN LEUKEMIC CELL LINE K562-ADM", CANCER, vol. 68, no. 8, 1991, pages 1714-1719, XP002587817, ISSN: 0008-543X
- TSURUO T ET AL: "EFFECTS OF QUINIDINE AND RELATED COMPOUNDS ON CYTOTOXICITY AND CELLULAR ACCUMULATION OF VINCRISTINE AND ADRIAMYCIN IN DRUG-RESISTANT TUMOR CELLS", CANCER RESEARCH, AMERICAN ASSOCIATION FOR CANCER REREARCH, US, vol. 44, no. 10, 1 October 1984 (1984-10-01), pages 4303-4307, XP008038937, ISSN: 0008-5472
- PILLOZZI S ET AL: "HERG potassium channels are constitutively expressed in primary human acute myeloid leukemias and regulate cell proliferation of normal and leukemic hemopoietic progenitors.", LEUKEMIA : OFFICIAL JOURNAL OF THE LEUKEMIA SOCIETY OF AMERICA, LEUKEMIA RESEARCH FUND, U.K SEP 2002 LNKD- PUBMED:12200695, vol. 16, no. 9, September 2002 (2002-09), pages 1791-1798, XP002587818, ISSN: 0887-6924 cited in the application
- PILLOZZI S ET AL: "Chemotherapy resistance in acute lymphoblastic leukemia requires hERG1 channels and is overcome by hERG1 blockers", BLOOD, AMERICAN SOCIETY OF HEMATOLOGY, US, vol. 117, no. 3, 3 November 2010 (2010-11-03), pages 902-914, XP008134468, ISSN: 0006-4971, DOI: DOI:10.1162/BLOOD-2010-02-262591 [retrieved on 2010-11-03]
- ZENG ZHIHONG ET AL: "Inhibition of CXCR4 with the novel RCP168 peptide overcomes stroma-mediated chemoresistance in chronic and acute leukemias", MOLECULAR CANCER THERAPEUTICS, vol. 5, no. 12, December 2006 (2006-12), pages 3113-3121, XP008139367, ISSN: 1535-7163
- MANABE ATSUSHI ET AL: "Adhesion-dependent survival of normal and leukemic human B lymphoblasts on bone marrow stromal cells", BLOOD, vol. 83, no. 3, 1994, pages 758-766, XP002649432, ISSN: 0006-4971
- BURGER JAN A ET AL: "Blood-derived nurse-like cells protect chronic lymphocytic leukemia B cells from spontaneous apoptosis through stromal cell-derived factor-1", BLOOD, vol. 96, no. 8, 15 October 2000 (2000-10-15), pages 2655-2663, XP002649433, ISSN: 0006-4971
- KONOPLEVA M ET AL: "Stromal cells prevent apoptosis of AML cells by up-regulation of anti-apoptotic proteins", LEUKEMIA, MACMILLAN PRESS LTD, US, vol. 16, no. 9, 1 September 2002 (2002-09-01), pages 1713-1724, XP002424990, ISSN: 0887-6924, DOI: DOI:10.1038/SJ.LEU.2402608

## Description

The present description refers to pharmaceutical compositions for the treatment of chemoresistant or potentially chemoresistant leukaemias, protocols for treatment of said leukaemias with said compositions, *in vitro* methods for the screening of compounds suitable for use in the treatment of chemoresistant or potentially chemoresistant leukaemias.

### STATE OF THE ART

Leukaemia is a general term which encompasses a wide spectrum of diseases: a first distinction is between acute and chronic forms. Moreover, such pathological form can be divided into lymphoblastic (lymphoid) leukaemias and myeloid leukaemias. The combination of the two classification criteria leads to the identification of four main forms of leukaemia: Acute lymphoblastic leukaemia (ALL); Chronic lymphoid leukaemia (CLL); Acute myeloid leukaemia (AML); Chronic myeloid leukaemia. ALL is the most common form in children, but can affect also adults, older than 65 years. Standard treatments comprise chemotherapy and radiotherapy. Survival depends on patients' age: it is 70-80% in children, 50% in adults. CLL mainly affects adults, males, over 55 years old, it is never detected in children. Five years survival is around 75%. AML more frequently affects adults, more rarely children, and five years survival of this leukaemia form is around 40%. Also in this case, the treatment of choice is chemotherapy, and, in some cases, bone marrow transplantation. CML affects mainly adults, and only in a small percentage children. The treatment of choice includes a molecular targeted inhibitor, i.e. Imatinib (Gleevec), which inhibits bcr/abl protein. Five year survival is around 80%.

In the last years, acute leukaemia survival, and in particular pediatric ALL survival, has greatly improved. Despite this fact, in a high percentage of children and/or adults affected by this disease, recurrence still occurs. These failures of chemotherapy lead to the indication for bone marrow transplantation, a treatment that has a strong impact not only on the patient, but also on the health system, due to the high costs of transplants. The main cause of treatment failure is due to mechanisms of intrinsic or acquired drug resistance. It has been shown that bone marrow stromal cells provide a refuge for some leukaemic cell populations, in particular for those stem cell populations that are responsible for the development and maintenance of the leukaemic disease, and that can evade the apoptotic death induced by chemotherapy and can acquire a therapy resistant phenotype (Konopleva, M et al. Stromal cells prevent apoptosis of AML cells by up-regulation of anti-apoptotic proteins. Leukaemia 16: 1713-1724 (2002).

The mechanisms of protection mediated by stromal cells involve complex reciprocal interactions between stroma-derived factors, in particular the chemokine SDF-1α (stroma-derived factor 1α) and its receptor CXCR4 (Zeng, Z et al. Inhibition of CXCR4 with the novel RCP168 peptide overcomes stroma-mediated chemoresistance in chronic and acute leukaemias. Mol. Cancer Ther. 5: 3113-3121 (2006)). Another mechanism by which stromal cells confer protection to leukaemia cells, involves the interaction between adhesion receptors (in particular the integrin VLA4), expressed on leukaemia cells, and adhesion molecules, like fibronectin, present on the surface of marrow stromal cells (Tabe, Y et al. Activation of integrin-linked kinase is a critical prosurvival pathway induced in leukaemic cells by bone marrow-derived stromal cells. Cancer Res. 67: 684-694 (2007)). Recent data highlight how integrins can trigger cell survival signals, through the assembly of macromolecular complexes with different proteins present on the plasma membrane. One of the partners involved in these complexes is represented by ion channels. The channel protein is not only a passive interactor, but often feed backs by controlling integrin activation and downstream signalling (Arcangeli, A Becchetti, A. Complex functional interaction between integrin receptors and ion channels. Trends Cel.l Biol. 16: 631-639 (2006)). These mechanisms can provide a molecular confirmation to the recent demonstration that ion channels, mainly K⁺ channels, mark and regulate specific steps of neoplastic progression, and can hence represent novel targets for antineoplastic therapy (Arcangeli, A et al. Targeting ion channels in cancer: a novel frontier in antineoplastic therapy. Cur. Med. Chem. 16(1): 66-93 (2009)). Among ion channels, hERG1 channels (also known as KCNH2 or Kv11.1, Gene Bank AAH01914.2), encoded by the *ether-a-gò-gò-related gene 1*, represent a relevant example of how this type of K⁺ channel can form multiprotein complexes on the plasma membrane of tumour cells (Arcangeli, A Becchetti, A. Complex functional interaction between integrin receptors and ion channels. Trends Cel.l Biol. 16: 631-639 (2006)). Moreover, hERG1 channels represent an important pharmacological target for antineoplastic therapy, as already hypothesized and reported in the literature published by the inventors (5), and as demonstrated by the fact that drugs that can inhibit hERG1 (that we shall call from now on "hERG1 blockers") inhibit neoplastic growth and progression, both *in vitro* and *in vivo* (Arcangeli, A et al. Targeting ion channels in cancer: a novel frontier in antineoplastic therapy. Cur. Med. Chem. 16(1): 66-93 (2009).

Pillozzi S et al. 2002 (Pillozzi S., et al. HERG potassium channels are constitutively expressed in primary human acute myeloid leukaemias and regulate cell proliferation of normal and leukaemic haemopoietic progenitors. Leukaemia, 16, 1791-1798, (2002)) and Pillozzi S et al. 2007 (Pillozzi S, et al. VEGFR-1 (FLT-1), β1 integrin and hERG K+ channel form a macromolecular signaling complex in acute myeloid leukaemia: role in cell migration and clinical outcome. Blood, 110: 1238-1250, (2007)), in addition to the already cited Arcangeli A et al., 2009 (Arcangeli, A et al. Targeting ion channels in cancer: a novel frontier in antineoplastic therapy. Cur. Med. Chem. 16(1): 66-93 (2009)), highlight the important role of the hERG1 channel in the leukaemic disease. In the first paper it is described how hERG1 activity is related to proliferative mechanisms and cell cycle progression, both in AML cell lines and primary samples. Unlike normal haematopoietic progenitor cells, hERG1 is constitutively expressed in leukaemic cells and is necessary for their progression into the cell cycle. The cited papers show how the administration of "hERG1 blockers" (class III antiarrhythmic drugs, like E4031 or Way 123,398), impairs cellular growth.

In the second publication the presence and the role of a macromolecular complex comprising, in addition to the hERG1 channel, the receptor of type 1 (FLT-1) for the angiogenetic factor VEGF-A, and the adhesion receptors of the integrin family, both in AML leukaemia cell lines, and in leukaemic blasts of different FAB, isolated from patients affected by leukaemia have been described. Moreover, data were provided to show how the activation of FLT-1 is able to induce an intense migratory and invasive activity in AML cells, and how this effect depends on the formation of the signalling complex. In particular, the correct activity of hERG1 channel was crucial to trigger the signalling activity of the receptor FLT-1 and the consequent migration of leukaemia cells.

The association (both physical and functional) between FLT-1, hERG1 and β1 was also demonstrated in primary AML blasts, in not only the co-expression of genes *herg1* and *flt-1,* but also the co -immunoprecipitation of the three membrane proteins involved in complex formation was demonstrated.

In addition, from the studies performed it emerged that the presence of the FLT-1 receptor with the functional hERG1 channel confers to leukaemic blasts a pro-migratory phenotype. Finally, experiments were conducted on immunodeficient mice inoculated with leukaemic cells, showing that the over-expression of hERG1 induces leukaemic blasts to exit from peripheral blood and hence to invade extramedullary sites.

In other words, the functional expression of the hERG1 channel on the plasma membrane confers a selective advantage to leukaemic blasts, that acquire a higher capacity to leave the bone marrow microenvironment and to enter the bloodstream.

Overall, in the cited paper, it has been demonstrated that the hERG1 channel is able to regulate various physio-pathological aspects of AML, such as the survival and cell proliferation within the bone marrow, and the increase in motility and trans-endothelial migration. The authors believe that, taken together, these effects may be responsible for the increased malignancy of herg1 + blasts, demonstrated *in vivo* in a cohort of patients with AML.

Pillozzi et al, Blood 2007, 222a "Expression and role of hERG1 channels in paediatric acute lymphoblastic leukaemias: shortcoming of drug resistance by hERG1 channel inhibitors in stroma-supported leukaemia cell cultures in vitro" teaches that targeting of hERG1 channels can restore proapoptotic response to chemotherapy in resistant B lymphoid leukaemic cells.

Pillozzi et al, Blood 2008 575-576 "A macromolecular signalling complex formed by CXCR4, VLA4 and hERG1 K+ channels mediates bone marrow-induced chemo-resistance" discloses that B-ALL leukaemia cell lines, when cultured on stromal cells of the bone marrow (BMSC) experience strong reduction in apoptosis after treatment with chemotherapeutic drugs such as doxorubicin, prednisone, L-asparaginase, metothrexate and that the addition of hERG1 blockers bypasses drug resistance.

Finally, in the publication Arcangeli, A et al. "Targeting ion channels in cancer: a novel frontier in antineoplastic therapy." Cur. Med. Chem. 16(1): 66-93 (2009), the authors have provided different experimental evidences not only to the fact that hERG1 plays a fundamental role in the control of different aspects of neoplastic progression, both in leukaemias and in human solid cancers, but also to the fact that it represents an excellent pharmacological target for antineoplastic therapy. In fact, in the literature, but also on the pharmaceutical market, many drugs exist capable of blocking the hERG1 channel (the "hERG1 blockers"). In the publication (5) the authors show numerous evidences, both *in vitro* and *in vivo,* that some "hERG1 blockers" (in particular the class III anthyarrhythmics, like E4031 and Way 123,398) behave as potential antineoplastic drugs.

The need to identify drugs and therapies that allow to treat effectively chemoresistant leukaemias is clear.

### SUMMARY OF THE INVENTION

The invention of the present description lies on the discovery that chemoresistance in many different forms of leukaemia is removed or strongly reduced by a treatment with at least one compound for cancer therapy in combination with at least one blocker of hERG1, that exert, with an unexpected synergic effect, a proaoptotic effect on chemoresistant leukaemic cells.

The present invention thus refers to:
- new combinations of a blocker of hERG1 channels which is erythromycin and one or more compound for cancer therapy selected in the group: doxorubicin, cortisone-based compounds, wherein said cortisone-based compound is selected in the group: prednisone, prednisolone, dexamethasone, betamethasone, cortisol, for use in the treatment of chemoresistant and/or potentially chemoresitant leukaemias.
- pharmaceutical compositions comprising at least a compound for cancer therapy selected in the group: doxorubicin, cortisone-based compounds, wherein said cortisone-based compound is selected in the group: prednisone, prednisolone, dexamethasone, betamethasone, cortisol, in combination with an inhibitor of hERG1 potassium channel, which is erythromycin for use in the treatment of chemoresistant and/or potentially chemoresitant leukaemias

The present disclosure describes also:
- an *in vitro* method of co-culture of leukaemic cells with marrow stromal cells comprising the step of culturing marrow stromal cells and subsequently adding leukaemic cells from leukaemia cell line or primary leukaemic cells.
- said cellular co-culture : and
- an *in vitro* method of screening for the identification of compounds or combinations of compounds effective in the treatment of chemoresistant leukaemias and /or potentially chemoresistant leukaemias comprising such steps:
   a) treating said cellular co-culture as previously defined wherein said leukaemic cells are chemoresistant, with one ore more compound or combination of compounds;
   b) evaluating the apoptosis of leukaemic cells after the treatment;
   c) identifying the compounds or combinations of compounds effective in the recovery of apoptotic pathways.

### DETAILED DESCRIPTION OF THE FIGURES

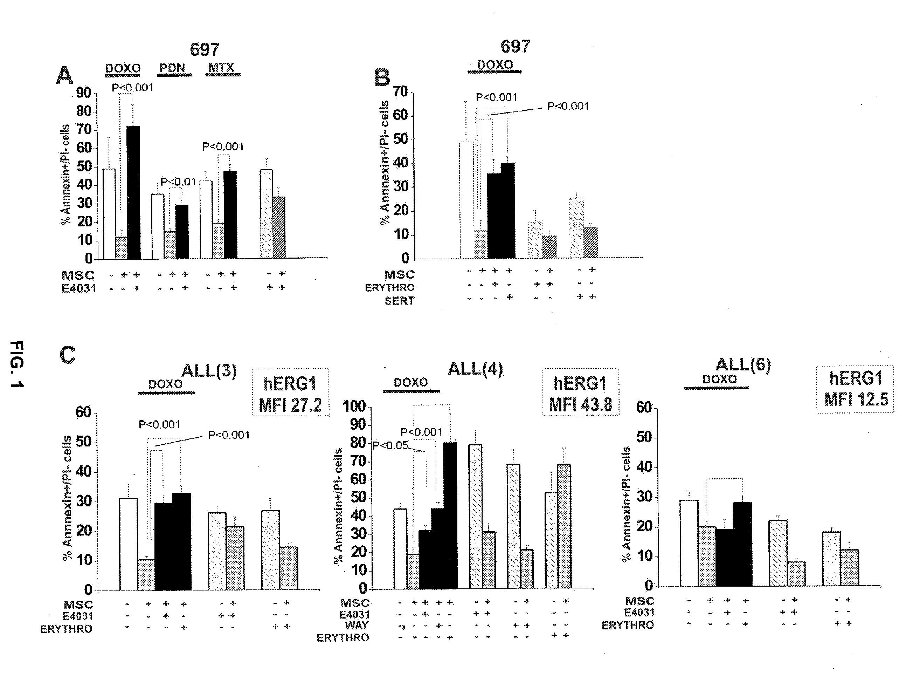

**Figure 1**. Effect of hERG1 inhibitors on apoptosis induced by Doxorubicin in human leukaemic cells, cultured *in vitro* in the absence or in the presence of marrow stromal cells (MSC), and treated with E4031, Erythromycin, Sertindole and Way (indicated with +) or not treated (indicated with -). A, B) 697 cells; C) Cells ALL(3), ALL(4) e ALL(6). Aliquots of 1x10⁵ cells have been centrifuged at 1100 rpm for 5 minutes (min) and washed in PBS 1X before incubation at room temperature for 15 minutes with the primary antibody (murine monoclonal anti hERG1 antibody directed against an extracellular portion of the channel, diluted 1:50). At the end of incubation, several washes in PBS 1X are performed, followed by a 15 min incubation with the secondary antibody (anti-mouse IgG FITC (1 µg/10⁶cells)). The pellet is washed twice in PBS and resuspended in 500 µl of a solution of 1% formalin in PBS. The analysis is performed with the flow cytometer FACScan (*Beckon Dickinson*). A protocol that allows to express in absolute quantitative terms the expression of hERG1 at the level of the cell plasma membrane, regardless of the flow cytometer used, of the voltage of photomultiplier and of the user has been standardised. The detection of the antigen analysed is obtained by using fluorescent tracers that generate a signal which is translated in terms of intensity of fluorescence. The Mean Fluorescence Index (MFI) is defined as the ratio between the mean fluorescence of the sample and the fluorescence detected in a sample incubated with the secondary antibody (that is fluorescent and hence the sample shows a signal which is not due to the direct antigen-antibody binding). In other words, such index can be calculated as the ratio between the specific signal of the sample which has been labelled with both the primary and secondary antibody (mean fluorescence of the sample = FC) and the aspecific signal obtained in the same sample labelled only with the secondary antibody (mean fluorescence of the control = Fc), multiplied by 100: hence MFI = FC/Fc 100. The values obtained, expressed in a percentage scale, provide an estimate which is independent from the fluorochrome linked to the secondary antibody (Cy3, Cy5, Alexa 488, PE, besides FITC can be used) and from the flow cytometer which has been used.

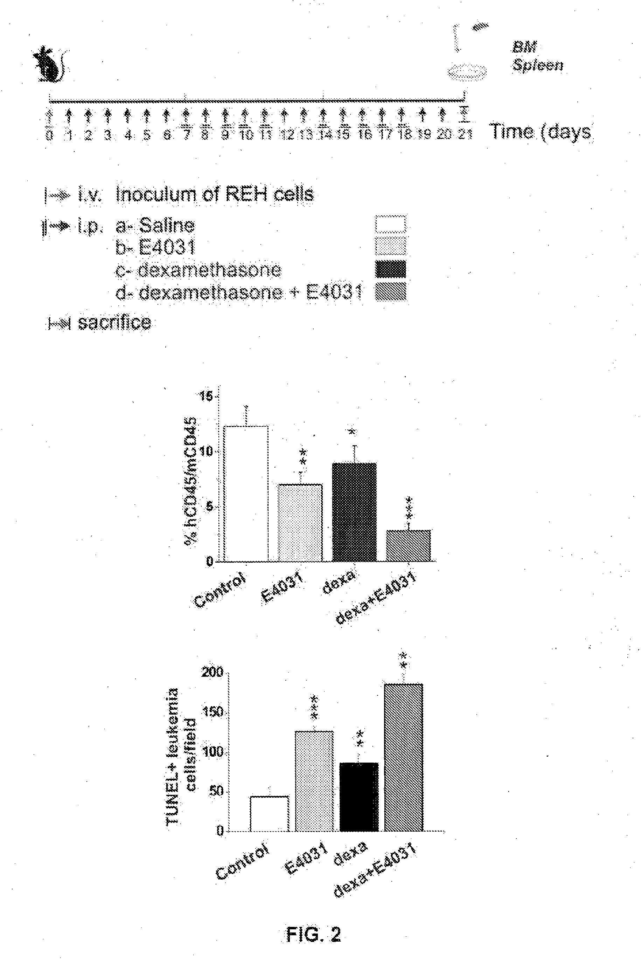

**Figure 2**. Effect of corticosteroids, inhibitors of hERG1 and of the combination corticosteroids/hERG1 inhibitors in an *in vivo* model of human leukaemia disease (human leukaemic cells REH inoculated into NOD/SCID mice). Cells were inoculated by injection into the caudal vein and seven days after the inoculum, animals were treated with E4031, Dexamethasone, Dexamethasone + E4031 for two weeks; control mice were treated, with the same schedule, solely with saline solution. At the end of the two weeks of treatment, animals were sacrificed and the spleen and bone marrow were removed. Bone marrow engraftment was evaluated by measuring the percentage of hCD45⁺ cells *versus* mCD45⁺ cells by flow cytometry. For the evaluation of apoptosis, the Tunel assay was performed on the histological sections of the treated animals.

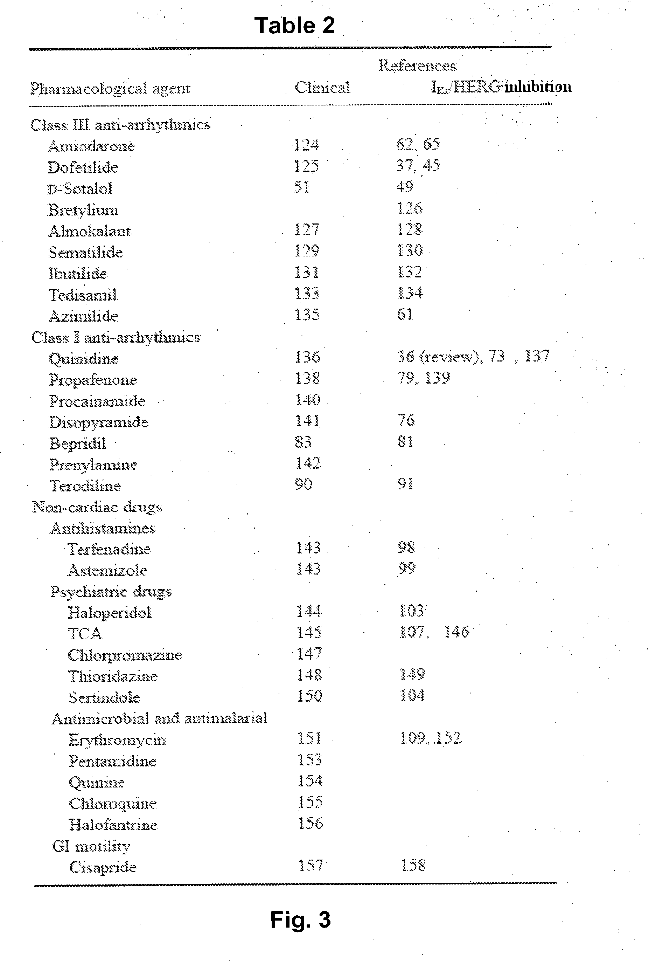

**Figure 3****.** In this figure, the Table 2 from pag 759 of the paper of Witchel HJ and Hancox JC- Familial and acquired Long QT syndrome and the cardiac rapid delayed rectifier potassium current- Clin Exp Pharmacol Physiol 27, 753-766, 2000, is reported. The table shows some active ingredients that can be selected as hERG1 channel blockers hERG1 and references to clinical trials or on their molecular hERG1 channel blocking activity suitable for the execution of the mixture or of the compositions of the present description.

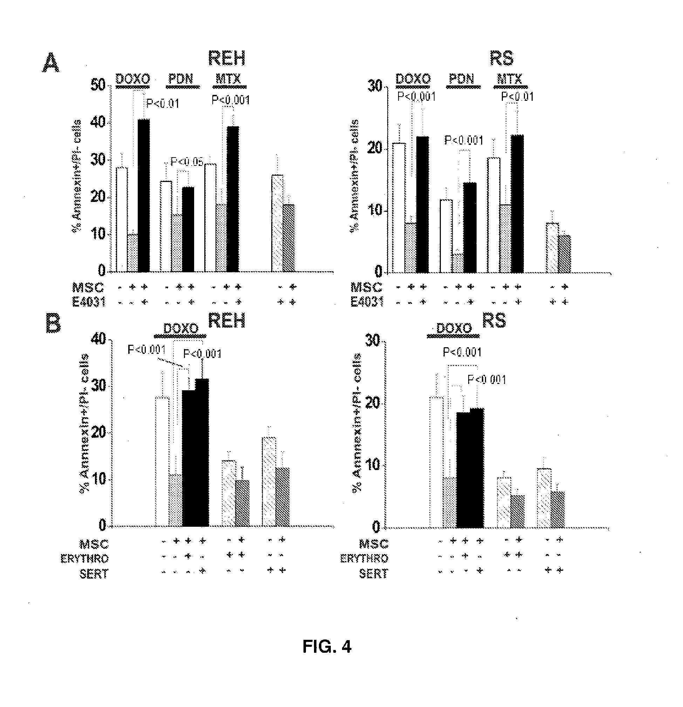

**Figure 4****.** hERG1 activity regulates the chemoresistance induced by stromal cells in leukaemic cells a). REH e RS4;11 cell lines were cultured in the presence or in the absence of MSC and treated with doxorubicin (0.1µg/ml), prednisone (5 µM) o methotrexate (1.5 µM), in the presence or in the absence of the hERG1 inhibitor E4031 (20 µM) for 48 hours. The percentage of Annessin V⁺/PI⁻ cells was evaluated after 48 hours of culture. b) REH and RS4; 11 cell lines were treated with doxorubicin (0.1 µg/ml) in the presence or in the absence of hERG1 inhibitors erythromycin (100 µM) or sertindole (1 µM). The percentage of Annexin V + / PI-cells was evaluated after 48 hours of culture.

### GLOSSARY

**hERG1**: "hERG1" indicates the potassium channel encoded by the human gene KCNN2 (or Kv11.1, Gene Bank AAH01914.2), in all its possible isoforms; where "hERG1" is indicated in the text, can be read also as "the mammalian homologue" of hERG1.

**Leukaemias**: The term leukaemias, in the present description, as in the medical environment, means a group of malignant diseases characterized by an uncontrolled, hence neoplastic, proliferation of hematopoietic cells.

The definition **"potentially chemoresistant leukaemia"** indicates leukaemic forms in which predictive methods show that the leukaemic form analyzed has the potential to develop chemoresistance.

**Chemoresistance:** In the present description, the term chemoresistance, or chemio resistance or more generally drug-resistance, means a reduction or a lack of responsivity of tumour cells to a particular drug, such as, by way of example, a chemotherapeutic drug or a drug used in antitumour therapies, used alone or in combination, with other drugs commonly used in antineoplastic therapies.

**MFI:** indicates the mean fluorescence intensity of the sample labelled a primary anti-hERG1 antibody and then with a fluorochrome-labelled secondary antibody against the primary antibody, compared to the fluorescence intensity of a sample of the same cells labelled only with the fluorochrome-labelled secondary antibody. In this particular case, it is an indirect, semi-quantitative, index of the amount of hERG1 protein present on the plasma membrane of the cells under study.

As a **"typical dosage of anticancer compounds in cancer therapy"** the dose typically used as a unit dose in the standard medical protocols or indicated in the accompanying documentation of the packages of anticancer medicinal products known to the skilled person is intended. This value, instantly recognizable by the skilled person, that varies from drug to drug, cannot be defined in a way applicable to all the commonly used anticancer agents without being defined as herein indicated.

The definition, applied to any anticancer drug, indicates exactly the dosage commonly prescribed and specified in the medical and pharmacological protocols for cancer therapy for each anticancer drug known to the skilled person. In the description below several examples of such values are reported.

### Therapeutic treatments

### a) pediatric forms

**Acute lymphoblastic leukaemia (pediatric)**

| | **drug** | **dose** |
|---|---|---|
| INDUCTION PRE-PHASE | | |
| | **PREDNISONE** | 40-60mg/mq/die os |
| | **METHOTREXATE** | 12 mg (≥3 years old) intrathecal; 3.3 mg /mq im |
| INDUCTION PHASE 1A | | |
| | **DEXAMETHASONE** | 10mg/mq/die p.o. |
| | **VINCRISTINE** | 1.5 mg/mq iv |
| | **DAUNOBLASTINE** | 30 mg/mq/dose iv |
| | **DOXORUBICIN** | 60-75 mg/mq iv |
| | **L-ASPARAGINASE** | 5.000-6000 Ul/mq/dose i.m. |
| INDUCTION PHASE 1B | | |
| | **CYCLOPHOSPHAMIDE** | 1000 mg/mq/dose iv |
| | **6-MERCAPTOPURINE** | 60 mg/mq/die p.o. |
| | **CYTOSINE ARABINOSIDE** | 75 mg/mq/die s.c. or iv |
| | **METHOTREXATE** | 12 mg (≥3 years old) intrathecal |
| CONSOLIDATION | | |
| | **6-MERCAPTOPURINE** | 25 mg/mq os |
| | **CITROVORUM FACTOR** | 7,5 mg/mq iv |
| RE-INDUCTION | | |
| IIa | **DEXAMETHASONE** | 10 mg/mq/die os |
| | **VINCRISTINE** | 1.5 mg/mq iv |
| | | |
| | **ADRIAMYCIN** | 30 mg/mq/dose iv |
| | **L-ASPARAGINASE** | 10.000 Ul/mq/dose i.m. |
| IIb | **CYCLOPHOSPHAMIDE** | 1000 mg/mq iv. |
| | **6-THIOGUANINE** | 60 mg/mq/die p.o. |
| | **CYTOSINE ARABINOSIDE** | 75 mg/mq/die s.c. or e.v. |
| MAINTENANCE | | |
| | **6-MERCAPTOPURINE** | 25 mg/mq os |
| | **METHOTREXATE** | 20 mg/mq p.o |

**Acute myeloid leukaemia (pediatric)**

| | **drug** | **dose** |
|---|---|---|
| PRE-phase | | |
| | **CYTOSINE ARABINOSIDE** | 40mg/mq/die iv |
| INDUCTION | | |
| | **IDARUBICIN** | 10mg/mq/die iv |
| | **ETOPOSIDE** | 100mg/mq iv |
| | **CYTOSINE ARABINOSIDE** | 20-70 mg /mq/die iv |
| CONSOLIDATION | **CYTOSINE ARABINOSIDE** | 20-70mg/mq iv |
| | **ETOPOSIDE** | 125mg/mq/die iv |
| CONDITIONING | | |
| | **BUSULFAN** | 16mg/Kg/die iv or os |
| | **MELPHALAN** | 140mg/mq/die iv |
| | **CYCLOPHOSPHAMIDE** | 60mg/Kg/die iv |

### b) adult forms

**Acute lymphoblastic leukaemia**

| | **DRUG** | **dose** |
|---|---|---|
| INDUCTION | | |
| | **VINCRISTINE** | 1.4 mg/mq iv |
| | **DOXORUBICIN** | 60-75 mg/mq iv |
| | **CYTARABINE** | 100 mg/mq iv |
| | **ETOPOSIDE** | 60-120 mg/mq iv |
| | **METHOTREXATE** | 2-20 mg/WEEK ir |
| | **CYCLOPHOSPHAMIDE** | 4-6 mg/kg iv |
| | **PREDNISONE** | 30-60 mg/die os |
| | **ASPARAGINASE** | 6000 u/mq i.m |
| CONSOLIDATION | | |
| | **ARA-C** | 2000 mg/mq x 2 iv. |
| | **VP-16** | 150 mg/mq iv. |

**Chronic lymphatic leukaemia**

| | **DRUG** | **dose** |
|---|---|---|
| INDUCTION/CONSOLIDATION | **CYCLOPHOSPHAMIDE** | 300 mg/mq iv |
| | **PREDNISONE** | 40 mg/mq os |
| | **VINCRISTINE** | 1 mg/mq iv |
| | **DOXORUBICIN** | 25 mg/mq iv |
| | **FLUDARABINE** | 25 mg/mq iv |
| | **BENDAMUSTINE** | 110 mg/mq iv |
| | **RITUXIMAB** | 375 mg/mq iv |
| | **ALEMTUZUMAB** | 3-30 mg /die iv |

**Acute myeloid leukaemia (except M3)**

| | **drug** | **dose** |
|---|---|---|
| INDUCTION | | |
| | **DAUNOROBICIN** | 60 mg/mq iv |
| | **CYTARABINE** | 100 mg/mq iv |
| | **PREDNISONE** | 40 mg/mq os |
| | **6-TIOGUANINE** | 70 mg/mq iv |
| | **HYDROXYUREA** | 20-30 mg/Kg/die os |
| CONSOLIDATION | | |
| | **CYTARABINE** | 500 mg/mq iv |

**M3**

| | **drug** | **dose** |
|---|---|---|
| INDUCTION | | |
| | **IDARUBICIN** | 12 mg/mq iv |
| | **ATRA** | 45 mg/mq po. |
| | **METHYLPREDNISOLONE** | 25 mg/mq os |

**Chronic myeloid leukaemia**

| | **drug** | **dose** |
|---|---|---|
| CRONIC PHASE | | |
| | **HYDROXYUREA** | 500-2000 mg/die/ os |
| | **INTERFERON ALFA** | 3-5 milioni U/mq/die sc |
| | **IMATINIB** | 400 mg/die os |
| | **CYTARABINE** | 100 mg/mq/die iv |
| ACCELERATED PHASE | | |
| | **IMATINIB** | 600-800 mg/die os |
| BLASTIC PHASE | | |
| | **INTERFERON ALFA** | 3-5 milioni U/mq/die sc |
| | **HYDROXYUREA** | 40 mg/Kg/die iv |
| | **6-MERCAPTOPURINE** | 0.5-1 mg/Kg/die po |

### Chemical name of hERG1 blockers

**E4031**
   N-[4 - [[1 - [2 - (6-Methyl-2-pyridyl) ethyl]-4-piperidinyl] carbonyl] phenyl] metanesolfonammide dihydrochloride
**SERTINDOLE**
   1 - (2 - {4 - [5-chloro-1-(4-FLUORO-PHENYL)-1 H-indole-3-IL]-IL-1-Piperidine}-ethyl)-imidazolidin-2-ONE, 1 - [2 - [4 - [5-Chloro-1-(4-fluorophenyl)-1H-indole-3-a]-1-piperidinyl] ethyl]-2-Imidazolidinone
**ERYTHROMYCIN**
   6 - (4-dimethylamino-3-hydroxy-6-methyl-Ossan-2-yl) oxy-14-ethyl-7,12,13-triidrossi-4-(5-hydroxy-4 methoxy-4 ,6-dimethyl -Ossan-2-yl) oxy-3, 5,7,9,11,13 - esametil ossaciclotetradecan-1-2,10-dione
**WAY 123,398**
   N-metil-N-(2-(metil(1-metil-1H-benzimidazol-2-il)amino)etil) ((metilsolfonil)amino)benzensolfonammide HCl

**AMIODARONE**
   (2 - {4 - [(2-methyl-1-benzofuran-3-yl) carbonyl]-diiodiofenossi -2.6} ethyl) diethylamine
**DOFETILIDE**
   N-[4 - (2 - {[2 - (4-metansulfonamide phenoxy) ethyl] (methyl) amino} ethyl) phenyl] metasulfonamide
**BRETYLIUM**
   N-(2-bromo benzyl)-N, N-dimethylethanolamine
**SEMATILIDE**
   N-(2-diethylaminoethyl)-4-benzamide-metansulfonamide
**IBUTILIDE**
   *N*-(4-{4-[ethyl(eptil)amino]-1-idrossibutil}fenil)metasulfonamide
**TEDISAMIL**
   3,7-bis(clclopropilmetil)spiro[3,7-diazabiciclo[3.3.1]nonane-9,1'-ciclopentano
**QUINIDINE**
   (S)-[(4S,5R,7R)-5-etinil-1-azabiciclo[2.2.2]octan-7-yl]-(6-metossiquinolin-4-yl)metanolo
**PROPAFENONE**
   1-[2-(2-hidrossi-3-propilaminopropossi)fenill]-3-fenilpropano-1
**PROCAINAMIDE**
   4-amino-N-(2-diethilaminoethil)benzamide
**DISOPYRAMIDE**
   N-(piperidin-2-ylmethyl)-2,5-bis(2,2,2-trifluoroethoxy)benzamide
**TERODILINE**
   N-terz-butll-4,4-di(fenil)butan-2-amina
**TERFENADINE**
   *RS*)-1-(4-*tert*-butylphenyl)-4-{4-[hydroxy(diphenyl)methyl]piperidin-1-yl}-butan-1-ol
**HALOPERIDOL**
   4-[4-(4-clorofenil)-4 idrossipiperidinA-1-il]-1-(4-fluorofenil)-butano-1
**CISAPRIDE**
   4-amino-5-chloro-*N*-((3*S*,4*S*)-1-[3-(4-fluorophenoxy)propyl]-3-methoxypiperidin-4-yl)-2-methoxybenzamide
**CHLOROQUINE**
   *N'*-(7-chloroquinolin-4-yl)-*N,N*-diethyl-pentane-1,4-diamine
**HALOFANTRINE**
   3-(dibutylamino)-1-[1,3-dichloro-6-(trifluoromethyl)phenanthren-9-yl]propan-1-ol
**TIORIDAZIN**
   *10-{2-[(RS)-1-Methylpiperidin-2-yl]ethyl}-2-methylsulfanyl-phenothiazine*
**PENTAMIDINE**
   4,4'-[pentane-1,5-diylbis(oxy)]dibenzenecarboximidamide

### DETAILED DESCRIPTION OF THE INVENTION

Subject-matter which is not encompassed by the scope of the claims does not form part of the claimed invention.

As detailed in the Section "state of the art", a major cause of the failure of conventional chemotherapy in the treatment of leukaemia, especially acute, lymphoid and myeloid forms lies in the fact that these forms can develop chemoresistance.

One of the most important cause of chemoresistance lies in the protective effect exerted by the bone marrow microenvironment, and in particular by bone marrow stromal cells.

It should be noted that, within the bone marrow, leukaemic stem cells, from which originate the leukaemic cells that invade the peripheral circulation, reside, survive and proliferate. It is precisely the leukaemic cells that reside in the bone marrow that must be eliminated by chemotherapy, in order to avoid the leukaemia disease, but it exactly is in the bone marrow that a mechanism of protection may develop, which prevents the destruction, or rather the induction of apoptotic death, of leukaemic cells.

In the present description an *in vitro* method developed by the authors that mimics the situation that occurs *in vivo* in chemoresistant leukaemias is disclosed comprising co-culturing leukaemic cells and bone marrow stromal cells. This method can be carried out by using resistant and not resistant leukaemic cell lines or primary leukaemic cells from biological samples.

The biological sample from which the leukaemia cells can be obtained, may be any sample from a patient affected by leukaemia from which said cells can be isolated; in particular, it will be possible to use, for example, blood and tissue samples. By way of example and not limiting to the purposes of this description, the tissue samples that can be used are fresh or frozen samples obtained from bone marrow aspirates, peripheral blood and lymph node biopsies of patients with leukaemia.

Biological samples obtained, irrespective of their nature, can be used either fresh or not. The skilled person, does not need, in this description, information on the various techniques of conservation of biological material as he/she will be able to choose without any inventive activity effort the most suitable to his needs, taking into account the detailed information, described in any laboratory manual.

The isolation of leukaemic cells, from the pool of different cell types present in the biological sample, can be carried out using anyone of the molecular characteristics that define biochemically said leukaemic cells. The leukaemic cells can be isolated by density gradient centrifugation (Abrams Ra, et al. Ficoll-Hypaque separation of bone marrow cells. Blood, 1985 66: 472-473). In general any method known to the skilled person to isolate from a pool of cells the population of leukaemic cells to be added to stromal cells in the co-culture it is considered appropriate for the purposes of this description. The bone marrow stromal cells can be obtained by techniques known to the skilled person, for example can be isolated and maintained in culture as reported in Manabe A. et al. Blood 1992 May 1;79(9):2370-7 pag. 2371.

The bone marrow stromal cells can be prepared by culturing in 96-well cluster mononuclear cells depleted of T cells isolated from the bone marrow of healthy donors, as described by Manabe and collaborators (Manabe et al, 1992).

Mononuclear cells can be isolated by density gradient centrifugation, washed several times with the medium RPMI-1640 and resuspended at the concentration of 2x10⁶/ml in Fischer medium with 5% FCS, 15% horse serum, transferrin (0.4 mg/ml), hydrocortisone (10⁻⁶ mol/l), L-glutamine (2 mmol/l), 2-mercaptoethanol (10⁻⁴ mol/l). Aliquots of 10 ml of cellular suspension are distributed in 25 cm flasks and 50% of culture medium is removed weekly. Cells become confluent after 4-6 weeks of culture.

The co-culture method herein described comprises the following step:
culturing marrow stromal cells in an adequate medium (AIM-V, Gibco) and subsequently adding to the culture leukaemic cells from primary leukaemic cells or from cell lines resuspended in the same medium.

Marrow stromal cells will be plated in multi well clusters, ranging from 50.000 to 100.000. After an incubation time of 12-24h at the conditions (37°C, 5% CO₂), leukaemic cells will be added (from about 80.000 to 100.000/well containing 50.000-100.000 stromal cells) and the cellular co-culture will be incubated for two hours at the conditions 37°C, 5% CO₂ before adding the compound or the combinations of compounds to test.

Any particular embodiment shown in detail will be in the following section "examples and experimental results". Any indication present in such section, can be applied to similar embodiments without needing any inventive step, by the skilled person.

Any data, present in the above mentioned section can also be applied, individually, to the present, detailed description.

The *in vitro* method as described above, mimics, in a surprisingly effective manner, the development of chemoresistance of leukaemic cells which occurs *in vivo.*

The method, applied on several samples of primary leukaemic cells, from patients whose clinical course is known, has shown the development of chemoresistance *in vitro* only in the co-cultures of primary leukaemic cells from those patients that have indeed developed a chemoresistant leukaemia, whereas no *in vitro* chemoresistance has been detected with the method described above in the cells from those patients whose leukaemic form has not developed chemoresistance.

A clear example of the functionality and effectiveness of the method is given by Figure 1, wherein in panel C, wherein primary leukaemic cells have been co-cultured: it is evident that for the first two samples free leukaemic cells are extremly sensitive to the drug (white bar: msc- E4031- eritro-) while in co-culture (grey bar: msc+ E4031- eritro-) the effect of the drug is virtually non-existent. Patients from which where derived said primary cells, have developed chemoresistance.

In the third sample, (ALL6), the presence of MSC cells exerts a slight protective effect with respect to cases ALL4 and ALL3, which reduces to less than 10% the efficacy of the drug on cells in co-culture. The patient from which said cells derived has not developed chemoresistance.

The method of the present description hence allows to mimic *in vitro* what will happen *in vivo*, and can be mainly used for two purposes, first, the screening of compounds or combinations of compounds which can reduce the chemoresistance and induce apoptosis of leukaemia cells; the second the prediction or monitoring of the development of chemoresistance in the leukaemic forms analysed. This diagnostic method, can also be incorporated in a therapeutic method involving the administration of effective amounts of compositions for the treatment of chemoresistant leukaemic forms or potentially chemoresistant in patients in need thereof, as this method allows to predict which patients will develop forms of chemoresistant leukaemia.

The present description also describes the co-culture cells obtainable by the method described above that may also be defined as "the co-culture" or "coculture".

This definition indicates the co-culture resulting from the method described above, to which some additional steps may be added, that will lead either to the method of drug screening described below, or to the method of diagnosis/treatment described below.

In order to carry out a screening of compounds or combination of compounds that can be used to treat chemoresistant and/or potentially chemoresistant leukaemias, it is herein disclosed a method that allows to test the effectiveness of substances or combination of substances that can be used for the treatment of chemoresistant and/or potentially chemoresistant leukaemias.

Using the method described above with leukaemic cells of patients, the authors of the invention have observed that the proapoptotic effect of anticancer compounds on leukaemic cells is completely abrogated when leukaemic cells are cultured in the presence of stromal cells and they also noted that such chemoresistance, induced by stromal cells in chemoresistant or potentially chemoresistant cells, is fully reverted, as reported in detail in the examples and in Figure 1 and further confirmed in *in vivo* experiments on mice (Figure 2), if the anticancer compound is used in combination with one or more blocker of the hERG1 channel.

Hence, the present description discloses an *in vitro* method of screening for the identification of substances or combination of substances effective in the treatment of chemoresistant and/or potentially chemoresistant leukaemias, comprising the following steps:
b) treating the cell co-cultures (leukaemic cells and marrow stromal cells) described above with one or more substance or combination of substances to be tested;
c) evaluating the apoptosis of leukaemia cells after treatment.

All the aspects of the co-culture method described above apply to the method of screening which is herein disclosed.

Step b) can also be described as treating the co-cultures obtained in step a) of the co-culture method described above with one or more substance or combination of substances to be tested.

"Substance or combination of substances effective in the treatment of chemoresistant and/or potentially chemoresistant leukaemias" means substance or combination of substances which have the effect of inhibiting from the beginning the development of chemoresistant leukaemic cells or to revert chemoresistance of leukaemic cells treated with these substances and to restore the apoptotic pathway.

In an embodiment the combinations of substances tested may show a synergistic effect wherein the sum of the therapeutic effects of each substance taken separately is less than the therapeutic action of the combination of said substances in the treatment of chemoresistant leukaemic cells or forms.

In another aspect, the screening method of the present description allows to identify new substances or new uses of known substances as anticancer agents that reduce the leukaemic chemoresistance.

The substances to test may be added to the co-culture at various concentrations and for different incubation times such as for example after about 24, 36, 48 hours of incubation at about 37°C. The concentrations and the times at which substances should be tested, can be determined by the skilled person without any inventive activity.

The value of apoptosis of leukaemia cells after treatment with the substances to test can be determined using various techniques known to the skilled person, such e.g. as flow cytometry methods.

In Flow cytometry methods, for example, the amount of leukaemic cells in apoptosis can be determined using antibodies or substances labelled with a fluorophore able to bind specific markers that indicate the status of apoptosis of the cell.

A suitable apoptosis marker such as phosphatidylserine may be detected by the binding of annexin V, labelled with a fluorophore such as fluorescein.

When the apoptosis marker is exposed also by necrotic cells, as in the case of phosphatidylserine, a second marker can be used to distinguish the two cell populations, such as for example propidium iodide which selectively binds necrotic cells.

All the products needed to read the results or some of these can be supplied in a kit for the screening of potentially suitable substances for use in the treatment of chemoresistant and/or potentially chemoresistant leukaemias.

All reagents or part of them can be aliquoted in one or more vials and can be supplied as a kit.

The same samples analyzed by the method described above were also analyzed using a method for measuring the expression levels of the channel hERG1 that allows to express in absolute quantitative terms the expression of hERG1 on the cell membrane of leukaemic cell samples. The analysis of these samples as described in detail in the examples and in the figure, shows a correlation between the effect of the combination of chemotherapeutic drug (or antitumoral) + hERG1 inhibitor and the levels of hERG1 expression. In particular, the higher is the expression level of hERG1 and the more significantly leukaemic cells respond to the combined treatment with the chemotherapeutic (or antitumoral) drug + hERG1 inhibitor.

The data obtained *in vitro* regarding the correlation between the expression of hERG1 and the responsiveness of leukaemic cells to chemotherapy were, until now, confirmed by clinical data of patients from which the samples were obtained: the development of chemoresistance *in vitro* was observed in patients who displayed a high expression level of hERG1 and for which the clinical course confirmed the effective clinical development of chemoresistance. These data therefore further support the efficacy of the combination chemotherapeutic drug (or antitumoral) + inhibitor of hERG1 for the use in the treatment of chemoresistant leukaemias.

It has been then discovered that the use of "hERG1 blockers", in combination with anticancer compounds or chemotherapeutic agents commonly used in the induction and/or maintenance of leukaemia, and non effective when used alone, against chemoresistant leukaemia, results in an unexpected synergistic effect of the two substances, which abrogates leukaemic chemoresistance.

This effect creates a further, definite, advantage of avoiding the use of transplantation, which nowadays remains the option of choice when facing a leukaemic form that does not respond to chemotherapy, or at least when facing disease forms which display early signs of lack of response.

It is important to remember that bone marrow transplantation presents many serious clinical consequences for the patient (not least the possibility of developing a severe graft versus host disease (that is the rejection of the graft versus host tissue), and represents a very heavy cost for the health service.

The invention relates to a mixture of a blocker of hERG1 channels which is erythromycin in combination with at least one compound for cancer therapy (defined here as the "antitumoral" in general) selected in the group: doxorubicin, cortisone-based compounds, wherein said cortisone-based compound is selected in the group: prednisone, prednisolone, dexamethasone, betamethasone, cortisol for the use in the treatment of chemoresistant and/or potentially chemoresistant leukaemias.

According to the present description, chemoresistant and/or potentially chemoresistant leukaemias mean all forms of leukaemia that can give chemoresistance or resistance to chemotherapeutic drugs commonly used in anti cancer therapies.

In the present description "potentially chemoresistant leukaemias" are define all the leukaemic forms in which predictive methods of such chemoresistance suggest that the leukaemic form analysed has the potential to develop chemoresistance.

A not restrictive example of chemoresistant and/or potentially chemoresistant leukaemic forms is selected in the group comprising adult and pediatric leukaemias such as pediatric or adult acute lymphoblastic leukaemia, adult and pediatric acute myeloid leukaemia, adult chronic lymphoblastic and myeloid leukaemia.

Acute lymphoblastic leukaemia is a type of malignant and progressive leukaemia, which mainly affects children younger than 15 years, whereas its incidence rate declines in adults. The classification of acute lymphoblastic leukaemia involves three different forms depending on the shape of the blasts.

Acute myeloid leukaemia (AML or LAM) in the medical field means a malignant neoplasm characterized by the proliferation of granulocytes. All embodiments of the present description are applicable to humans and also to mammals in general.

According to the present description any compound known to the skilled person able of blocking, reducing or inhibiting (slowing down or abolishing) totally or partially hERG1 channel activity (see Figure 3 as an example) is defined as a hERG1 channel blocker.

According to this description, said hERG1 channel blocker is selected in the group comprising active class III antiarrhythmic principles, active class I antiarrhythmics principles, antihistamines active principles, active principles for psychiatric disorders, anti-microbials active principles, active principles for gastrointestinal mobility.

It is obvious that all active ingredients that are part of these classes having a hERG1 blocking activity can be selected, while those active ingredients that are members of those classes, but have no blocking activity on hERG1 channels, cannot be selected.

In the present description the hERG1 inhibitors shown in Figure 3 are included. Said active ingredients of class III antiarrhythmics can be selected in the group comprising E4031, WAY 123.398, amiodarone, dofetilide, D-sotalol, bretilio, almokalant, sematilide, ibutilide, tedisamile, azimilide.

Said active ingredients of class I antiarrhythmics can be selected in the group comprising quinidine, propafenone, procainammide, disopyramide, pepridile, prenilamina, terodilina. Said active ingredients antihistaminic can be selected from terfenadine, astemizole. Said active ingredients for use in the treatment of psychiatric disorders may be selected from the group comprising haloperidol, tricyclic antidepressants, chlorpromazine, sertindole or thioridazine. Said antimicrobial active ingredients can be selected in the group comprising of erythromycin, pentamidine, quinine, cloroquine, halofantrine. Said active ingredients for the gastrointestinal mobility such as e.g. cisapride.

In figure 3 of the present description, corresponding to Table 2 on page 759 of the paper Witchel HJ and Hancox, JC Familial and Acquired Long QT syndrome and the cardiac rapid delayed rectifier potassium current-Clin Exp Pharmacol Physiol 27, 753-766, 2000, are reported some active ingredients that can be selected as blocker of hERG1 channel and references to clinical trials or molecular studies on their molecular hERG1 blocking activity suitable for the realisation of the mixtures or compositions of the present description.

Said compound for antitumoral therapies may be any compound suitable for the treatment of chemoresistant and/or potentially chemoresistant leukaemia selected in the group: doxorubicin, cortisone-based compounds..

Among the cortisone-based commonly used in cancer therapy, the skilled person can identify any suitable cortisone-based; this can include, for instance, the use of cortisol, prednisone, prednisolone, dexamethasone and betamethasone.

The mixtures can be prepared by mixing one or more blockers of hERG1 channel and one or more anticancer compounds using techniques known to the skilled person, without any particular technical and experimental difficulties and without any inventive activity.

A further embodiment relates to a pharmaceutical composition for use in the treatment of chemoresistant leukaemias and/or potentially chemoresistant leukaemias, comprising a mixture of a blocker of hERG1 channel which is erythromycin, in combination with at least one compound for cancer therapy as defined in the claims.

The pharmaceutical compositions described herein can be prepared according to techniques known to the skilled person either by using a combination of one or more hERG1 channels blockers in combination with one or more compounds for cancer therapies previously prepared or by mixing the individual active substances directly in the preparation of the composition. Of course, the compositions may be prepared using one or more of the inhibitors (blockers) of hERG1 as described above or a pharmaceutically acceptable salt thereof and one or more antitumoral compound (or drug) as described above or a pharmaceutically acceptable salt thereof.

These compositions may obviously comprise one or more pharmaceutically acceptable vehicles, diluents and/or excipients. The compositions can be in any form deemed appropriate by the skilled person, such as solid, semi-solid, liquid, granular, inhalation or aerosol inhalation.

The liquid forms may be appropriate forms for oral or systemic administration.

The pharmaceutical compositions described herein may be suitable for oral administration (e.g. buccal and sublingual administration), rectal, topical (sublingual, buccal or transdermal administration), vaginal, parenteral (e.g. subcutaneous, intramuscular, intravenous or intradermal), inhalation or nasal to a mammal such as a human being.

Pharmaceutical compositions suitable for oral administration can be capsules, tablets, pills, powders, granules, solutions or suspensions in aqueous or non-aqueous liquids, foam or beaten edible, liquid oil in water emulsions or liquid water in oil emulsions.

For example, for oral administration in capsule or tablet form, the compounds mentioned above may be combined with a non-toxic pharmaceutically acceptable inert carrier such as ethanol, glycerol, water and similar. There may also be present flavourings, preservative, colouring and dispersant agents.

The capsules can be prepared by filling gelatine casings with the compounds mentioned above. Lubricants and gliding as colloidal silica, talc, magnesium stearate, calcium stearate or solid polyethylene glycol can be added to the mixture before the actual filling. A disintegrating or solubilizing agent can be added to improve the availability of the medicament when the capsule is ingested. Moreover, when desired or necessary, binders, lubricants, disintegrating agents, colouring agents can also be incorporated into the compositions. Suitable binders include for example starch, gelatine, natural sugars such as glucose or beta-lactose, such as softening, natural and synthetic rubbers such as acacia, tragacanth or sodium alginate, carboxymethyl cellulose, polyethylene glycol, waxes and the like. Lubricants used in these forms include sodium oleate, sodium stearate, magnesium stearate, sodium benzoate, sodium acetate, sodium chloride, and the like. The disintegrating agents include for example starch, methylcellulose, agar, bentonite, xanthan gum and the like.

Tablets are formulated, for example, by preparing a mixture powder, as granules or semi-elaborated, adding a lubricant and a disintegrating agent and pressing in tablets. A powder mixture is prepared by mixing the compound comminuted in an appropriate manner with a diluent or base as described above and, optionally, a binder such as carboxy methylcellulose, an alginate, gelatine or polyvinylpyrrolidone, a retarding solutions such as paraffin, an absorption accelerator such as a quaternary salt and/or an absorption agent such as bentonite, kaolinite or dicalcium phosphate. The powder mixture can be granuled by wetting it with a binder such as syrup, starch paste, acadia mucilage or solutions of cellulosic or polymeric materials and forcing it through a partition. As an alternative to granulating, the powder mixture can be passed through the tablet and the result is imperfectly formed in semi-broken into granules. The granules can be lubricated to prevent sticking to the molds that form the tablets by the addition of stearic acid, stearate salt, or mineral oil. The lubricated mixture is then pressed into tablets.

Mixtures of one or more hERG1 channel blockers in combination with one or more compounds for cancer therapy of the present disclosure can also be combined with an freely flowing inert carrier and can be pressed into tablets directly without going through the steps of granulation or semi-elaboration. A transparent or opaque protective coating consisting of a sealing coating of shell, a coating of sugar or polymeric material and a shiny coating of wax can be provided. Colorurs may be added to these coatings in order to recognize different dosage units.

A pharmaceutical composition suitable for oral administration in the form of a tablet may include one or more pharmaceutically acceptable vehicles and/or excipients suitable for the preparation of formulations in tablets. Examples of such vehicles include lactose and cellulose. The tablet can also instead contain one or more pharmaceutically acceptable excipients such as binders, lubricants such as magnesium stearate, and/or disintegrating agents for tablets.

A pharmaceutical composition suitable for oral administration in capsule form can be prepared using encapsulation procedures. For example, pellets containing the active ingredient may be prepared using a suitable pharmaceutically acceptable vehicle and then be placed in a hard gelatine capsule. Alternatively, a dispersion or suspension may be prepared using any suitable pharmaceutically acceptable vehicle, such as an aqueous rubber or an oil and the dispersion or suspension can then be placed in a soft gelatine capsule.

The composition may be in unitary dose form such as a tablet or capsule for oral administration, for example, for oral administration to a human being. Where appropriate, dosage unitary formulations for oral administration may be micro encapsulated. The formulation can also be prepared to prolong or maintain the release by way of example by coating or by embedding particulate material into polymers, waxes or the like.

Liquids for oral use as solutions, syrups and elixirs can be prepared in the form of dosage units so that a given quantity contains a predetermined quantity of the compounds mentioned above. The syrups can be prepared by dissolving the compound in a suitably flavoured aqueous solution, while elixirs are prepared through the use of a non-toxic alcoholic vehicle. The suspensions may be formulated by dispersing the compound in a nontoxic vehicle. They can also be added solubilisers and emulsifiers such as ethoxylated isostearil alcohols and polyoxyethylene sorbitol ethers, preservatives, flavours such as peppermint oil or saccharin or other artificial sweeteners and the like.

Generally a liquid formulation consists of a suspension or solution of the compounds mentioned above in one or more pharmaceutically liquid suitable vehicles, such as an aqueous solvent such as water, ethanol or glycerine, or a non-aqueous solvent, such as polyethylene glycol or oil. The formulation may also contain a suspending agent, preservative, flavouring and/or dye.

Compositions suitable for parenteral administration may include sterile aqueous or non-aqueous solution for injection which may contain antioxidants, buffers, bacteriostatic and solutes which render the solution isotonic with the blood of the intended recipient, and aqueous or non-aqueous sterile suspensions which may include suspending and thickening agents.

A parenteral composition may include a solution or suspension of the compounds in a vehicle such as sterile water or a parenterally acceptable oil. Alternatively, the solution can be lyophilised; the lyophilised parenteral pharmaceutical composition can be reconstituted with a suitable solvent just prior to administration.

The formulations may be presented in single dose or multi-dose containers, for example, sealed ampoules or vials, and may be stored in lyophilised condition requiring only the addition of the sterile liquid carrier, for example water for injections, immediately prior to use. Extemporaneous injection solutions and suspensions may be prepared from powders, granules, lyophilized and sterile compresses.

In the case of parenteral administration, the composition may also be provided with the active ingredients in separate containers that can be suitably admixed according to the desired dosage taking into account the weight, age, gender and health status of the patient in need thereof.

Compositions for inhalation or nasal administration may conveniently be formulated as aerosols, drops, gels or dry powders.

The compositions suitable for nasal administration wherein the vehicle is a solid include a coarse powder having a particle size e.g. in the range from 20 to 500 microns which is administered so that the sniff is taken, i.e. by rapid inhalation through the nasal passage from a container of powder held close to the nose. Formulations suitable wherein the vehicle is a liquid, for administration as a spray or nasal drops, include aqueous or oily composition.

The compositions suitable for administration by inhalation include small particle powders or aerosol particles that can be generated by various types of dose pressurized aerosols, nebulizers or inhalers calibrated.

The aerosol formulations, such as for administration by inhalation, may include a solution or a fine suspension of the active substance in an aqueous or non-aqueous pharmaceutically acceptable solvent. The aerosol formulations may be presented in single or multiple dose quantities in sterile form in a sealed container, which may take the form of a cartridge or a refill for use in a nebulizer or inhaler device. The blocking drugs can be administered orally or by parenterally. The compositions herein described may be presented in the form of unit dose containing a predetermined quantity of one or more hERG1 channel blockers and one or more compounds for anti cancer therapies in order to facilitate the administration and the uniformity of dosage.

Examples of such unit dosage forms are tablets (including scored or coated tablets), capsules, pills, powders, injectable solutions or suspensions, teaspoon, tablespoon, and the like, and their multiple separate.

The exact dosage and frequency of administration will depend on the particular combination of hERG1 channel blockers and of compounds for anticancer therapy used, the particular condition to be treated, the severity of the condition to be treated, age, weight and the overall physical condition of the particular patient as well as on other medications that the patient is taking, as is well known to experts in the field. It is also clear that this quantity can be effectively lowered or increased depending on the responses of the treated patient and/or according to the evaluation of the physician prescribing the compounds of the present invention. The effective doses given here are therefore only indicative.

The composition described herein may include one or more of such inhibitors in a unitary dose between about 0.3 and about 100mg/Kg/die altogether, for example about 1, 5, 10, 20, 30, 40, 50, 60, 70, 80, 90 mg / kg / day.

A not limiting example of the dosages that can be used for hERG1 channel blockers above mentioned are as follows:
On the basis of *in vitro* and *in vivo* experiments the dosage for the administration of inhibitors of hERG1 in combination with anticancer compounds are in the range considered safe and effective based on the bioavailability of individual drugs.

The dosage of anticancer compounds can be, in the composition of the invention, a dosage such as that commonly used in the therapy against not chemoresiatant leukaemias per kilogram of body weight or a lower dose given the synergistic effect due to the presence of the hERG1 inhibitor.

In the compositions herein reported, the dosage unit of the active anticancer compound may be about 100%, about 90%, about 80%, about 70%, about 60%, about 50%, about 40%, about 30% or less than the unit dose commonly used in the therapy for leukaemia.

Unit dose means the dose that is administered to the patient each time, whether divided into several doses throughout the day, be it daily, or it at intervals of days.

Non-limitative examples of doses currently used for anticancer drugs above indicated, applicable in the percentages above reported to the pharmaceutical composition and its unit dose according to this description follows:

### Dosage

### Doxorubicin hydrochloride

intravenously: when Adriblastine is used as single antiblastic agent the recommended dose in adults is 60-75 mg/mq of body surface area, administered by intravenous injection (iv) at intervals of 21 days consistent with the bone marrow conditions.

The lower dose (60 mg/mq) is recommended for patients with reduced bone marrow reserve due to advanced age, prior therapies, or bone marrow tumour infiltration. The dose of 60-75 mg/mq can be administered in a single injection or divided into 2-3 consecutive days. Especially for paediatric patients, it has been suggested an alternative dose of 30 mg/mq/die iv for 3 consecutive days; such cycle is repeated every 4 weeks. The cumulative dose of intravenous Adriblastine, whatever the administration schedule, must not exceed 550 mg/mq body surface area (see Special warnings and precautions for use in the SPC).

Adriblastine is currently used extensively in polychemotherapy at usual doses of 25-50 mg/mq every 3-4 weeks in combination with other agents with myelosuppresive action at doses of 60-75 mg/mq when combined with other drugs with no bone marrow toxicity.

### Prednisone

It is a drug normally given orally: the attack therapeutic dose in adults of average weight corresponds to 20-30 mg per day. This initial dose is rapidly reduced in the space of time of one week to a maintenance dose ranging on average around 10 mg per day: lower doses may also be required relative to body weight and age of the patient. The maintenance dose should always be the least able to control the symptoms and it is still determined by the physician that when administering an inadequate dose, will witness the gradual recovery of the symptoms. The reduction in dosage should always be gradual.

For administration of high doses in particular haematological, dermatological, forms etc.. Tablets with unit dosage higher than 25 mg can be used. It is important to emphasize that the need for corticosteroids is variable and then the dosage should be individualized, taking into account the disease and patient response to therapy.

The used cortisone-based are also: cortisol, prednisone, prednisolone, dexamethasone and betamethasone

Further information on the dosage for the treatment of leukaemias are available to the skilled person without any use of inventive activity by the same and no particular difficulties in obtaining the information. The dosage may also simply be the one indicated on the package inserts of anticancer drugs listed above.

This description provides also a method for the treatment of chemoresistant and/or potentially chemoresistant leukaemias, which comprises the administration to patients in need thereof, of effective quantities of a composition as described herein.

Said forms of chemoresistant or potentially chemoresistant leukaemias can be selected in the group comprising adult and paediatric leukaemias such as paediatric or adult acute lymphoblastic leukaemia, the adult and paediatric acute myeloid leukaemia, adult chronic lymphoblastic leukaemia, adult chronic myeloid leukaemia.

In the method of treatment, the exact dosage and frequency of administration of the compositions will depend on the particular combination of hERG1 channel blockers and of compounds used for anticancer therapy, the particular condition to be treated, the severity of the condition to be treated, age, weight and the overall physical condition of the particular patient as well as on other medicaments the patient is taking, as it is well known to the experts in the field.

Some effective doses that can be administered are listed below:
One or more of said hERG1 inhibitors can be administered at a dosage unit between about 0.3 and about 100mg/Kg/die altogether, for example about 1, 5, 10, 20, 30, 40, 50, 60, 70 , 80, 90 mg/kg/day in combination with one or more anticancer compounds of which the dosage unit may be about 100%, about 90%, about 80%, about 70%, about 60%, about 50%, about 40%, about 30% or less than the unit dose commonly used in therapy for leukaemia.

The method for the treatment of chemoresistant and/or potentially chemoresistant leukaemias may be preceded by one or more steps of screening that are known to the skilled person, in order to determine whether a patient is suffering from a chemoresistant leukaemia.

In one embodiment this method of treatment comprises the following steps of screening to determine the presence of actual or potential chemoresistance:
a) incubating a sample x of primary leukaemic cells from a patient with a primary monoclonal antibody anti-hERG1, which is specific for the portion external to the membrane of said ion channel;
b) incubating with a fluorochrome-labelled secondary antibody, specific for the primary antibody, the cells obtained after the incubation of step a) and a sample y of primary leukaemic cells from the same patient, not previously incubated with the primary antibody;
c) evaluating the fluorescence of both samples by flow cytometry;
d) calculating the MFI value, determined as the ratio between the fluorescence detected on sample x and the fluorescence detected on sample y, if the MFI value is equal or higher than 25,
e) administering effective quantities.of a composition according to any of claims from 7 to 12.

The screening steps described herein are designed to predict whether leukaemic forms selected in the group comprising adult and paediatric leukaemias such as adult and paediatric acute lymphoblastic leukaemia, adult and paediatric acute myeloid leukaemia, adult chronic lymphoid leukaemia, adult chronic myeloid leukaemia.

The information regarding the prediction of the development and/or the monitoring of the time course of the progress of chemoresistance in time will be obtained by an analysis and elaboration of data obtained on the expression level of the ion channel hERG1 on the plasma membrane of leukaemic cells.

The leukaemic cells to be analyzed will be cells obtained by biological samples of leukaemic forms chosen in the group which comprises adult and paediatric leukaemias, as for example the acute lymphoblastic leukaemia, both paediatric and of the adults, the acute myeloid leukaemia, both paediatric and of the adults, the chronic lymphoid leukaemia of the adult and the chronic myeloid leukaemia of the adult.

The biological sample from which the leukaemic cells will be obtained, can be any sample from patients affected by leukaemia from which it is possible to isolate such cells; in particular, it will be possible to use, e.g., blood or tissue samples.

By way of example and not limiting the scopes of this description, the tissue samples to be used will be fresh or frozen samples obtained marrow aspirates, peripheral blood, from lymph node from leukaemic patients.

The biological samples may be used either fresh or not, irrespective of their nature. The skilled person does not need in this description any information regarding the different techniques for the storage of biological material since he/her will be able to choose, without any inventive activity effort, the most appropriate to his/her needs in view of the detailed information present in any lab manual.

The isolation of the leukaemic cells, from the pool of cells of different nature present in the biological sample, can be carried out exploiting any one of the molecular characteristics that define biochemically said leukaemic cells.

Leukaemic cells can be isolated by density gradient centrifugation (Ficoll-Hypaque (d = 1077 g/ml)) (as indicated in the protocol in RA Abrams"Ficoll-Hypaque separation of bone marrow cells". Blood 1985; 66:472:473). In general, it is considered appropriate for the purposes of this description any method known to the skilled person, which makes it possible to isolate from the whole cell population, a set of cells of the population of leukaemic cells to analyze.

In this description, the development of an index (MFI) directly related to the expression of hERG1 channels on the cells analyzed to predict whether patients from which the samples examined derive will develop or not chemoresistance (or drug resistance) during the course of the disease is disclosed. The MFI values then allow to know *a priori* the tendency to develop chemoresistance of the leukaemic form under analysis and provide useful tools for the physician to design an effective treatment. The hERG1 ion cannel, is a K⁺ channel, which, as shown above, is able to form multiprotein complexes on the plasma membrane of tumour cells and to regulate specific stages of neoplastic progression.

The hERG1 channel shows an extracellular portion of about 100 amino acids that may be used to identify recognition epitopes that can be used to develop antibodies. The extracellular portions are the S1-S2 portion, the S3-S4 portion, the S5-Pore portion and the S1-S2 portion is particularly suitable for the development of antibodies.

For the purposes of the present description, such epitopes will be used for the development of a monoclonal anti hERG1 antibody, following what is known to the skilled person.

In order to develop the monoclonal antibody, any standard technique for the development of primary antibodies will be sufficient: we recall here briefly that any specific antibody, which recognizes a specific antigenic determinant (epitope), is produced by a specific B lymphocyte. The isolation and culture *in vitro* of a cell capable of producing a single antibody represents the source of monoclonal antibodies (mono-specific). However, B lymphocytes, when cultured *in vitro,* die after a short time, and hence cannot be a source for the long term development of antibodies.

The technology of monoclonal antibodies comprises the isolation of these B lymphocytes and their subsequent fusion with transformed cells (myeloma cells), useful for their characteristics of higher proliferation and survival. Many of the resulting hybrid cells (or hybridomas), that are cultured *in vitro,* will maintain their immortality, in addition to producing large amounts of the monospecific antibody.

The fusion between B lymphocytes (obtained from the spleen and lymph nodes of an immunized animal) and the murine (the mouse is the most used animal specie) myeloma cells, is obtained trough the addition of a substance which promotes the fusion of the membranes, such as the polyethylenglycol.

The medium in which hybrids are cultured is a selective medium, known as HAT, that because of its composition, inhibits the growth of both myeloma and non fused spleen cells, but does not inhibit the growth of the hybridoma cells which complement the two parental cells.

Hybridomas are then submitted to a screening for the identification of the desired specific antibodies and those that are selected are then initiated to the storage or mass production.

Moreover, monoclonal anti-hERG1 antibodies, which are specific for epitopes present on the extracellular portion, are also available on the market (for example ALX-804-652-R300, from Alexis-Biochemical or ABIN195450 from Antibodies on-line) and can be used for the purposes of the present invention, without providing any further details in this description.

In particular, the concentration of antibody to use and also the incubation time of the leukaemic cells with the primary monoclonal antibody anti-hERG1 shall be sufficient, as well known to the skilled person, to guarantee the binding between the antigenic determinant and the antibody. Details relative to protocols of incubation with the antibody are well known to the technical skilled person and if commercially available antibodies are used, are indicated by the supplier.

For mere illustrative purposes, as reported in the examples described above, if the antibody which is utilized will be the one developed against the epitope comprised between residues 575-588 of the hERG1 channel (accession N° NM 000238 pubmed), it will be possible to catty out an incubation at room temperature for 15 min with an antibody dilution of about 1:50.

The detection of the primary monoclonal antibody, will be carried out by using a suitable specific fluorochrome-labelled secondary antibody that, as known, will have to be specific for the constant region, also known as Fc, of the primary antibody, which in turn depends on the animal species used for the development of the primary antibody itself.

In a particular embodiment in which the primary antibody is developed in the mouse, the secondary antibody, as well known to the skilled person, will be a secondary anti-mouse antibody.

The method here described is strictly related to the use of a flow cytometer as the inventors have found the possibility of expressing in absolute quantitative terms the expression of hERG1 at the level of the cellular plasma membrane, through this technique. For this reason, the secondary antibody shall be labelled with a fluorochrome. The secondary antibody may be labelled with any fluorochrome commonly used for the labelling of secondary antibodies and in particular it will be possible to use a fluorochrome selected in the group comprising: idrossicumarine, aminocumarine, metossicumarine, Cascade Blue®, Pacific Blue^{™}, Pacifc Orange^{™}, Lucifer Yellow, NDB, phycoerythrin (PE), PE conjugates, Texas Red®, Peridinin chlorophil (PerCP), TruRed, FluorX, fluorescein, BODIPY-FL, TRICT, X-rodamine, allophycocyanine (APC), APC conjugates, Alexa Fluor®, FITC, Cy3, Cy5, Cy2, Cy7.

The detection of the antigen of interest under exam can be obtained thanks to the fact that the fluorescent tracers generate a signal which is transduced for the first time in the present description in terms of median fluorescence intensity (MFI). The translation of the signal into MFI makes it possible to define a value that, independently of the flow cytometer employed, of the voltage of the photomultipliers and of the user, defines the expression level of the hERG1 channel on the plasma membrane.

The Mean Fluorescence Index (MFI) is defined as the ratio between the mean fluorescence of an aliquot of a sample incubated with a primary antibody and subsequently with a secondary, fluorochrome labelled, anti-primary antibody and the fluorescence of a control aliquot of the same sample incubated only with the secondary antibody (that is fluorescent and hence the sample shows a signal which is not due to the direct antigen-antibody binding, defined as "background"). In other words, such index can be calculated as the ratio between the specific signal of the sample labelled with a primary and a secondary antibody (mean fluorescence of the sample=FC) and the aspecific signal (background) obtained in the same sample labelled with only the secondary antibody (mean fluorescence of the control = Fc) and multiplied by 100: that is MFI= FC/Fc 100. The values obtained in this way, reported on a percentage scale, allow to have an assessment which is independent of the fluorochrome linked to the secondary antibody and of the flow cytometer utilized.

Studies performed by the inventors on leukaemia cells from patients affected by leukaemia, have shown how it is possible to establish a MFI value beyond which it is possible to predict the development of chemoresistance. In particular, the authors have developed a culture method *in vitro* that mimics, in a surprisingly effective manner, the development of chemoresistance in leukaemic cells which occurs *in vivo* that comprise the co-culture of leukaemic cells and stromal marrow cells. Such *in vitro* co-culture mimics the protective and the induction of chemoresistance effects observed in the bone marrow leukaemic cells and allows hence to under stand the molecular mechanism underlying this phenomenon.

It is evident in figure 1 C, leukaemic cells co-cultured with stromal cells are significantly less responsive to the treatment with doxorubicin compared to culture with leukaemic cells alone. The unresponsiveness to the chemotherapy drug, experimentally induced with the co-culture *in vitro,* is reverted with a therapeutic protocol that involves the combined administration of the chemotherapeutic drug and an inhibitor of the hERG1 channel. The MFI values calculated for the same chemoresistant leukaemia cells indicate that the more the MFI value is greater than 25 the more significantly leukaemia cells respond to the combined treatment with chemotherapeutic drug + hERG1 inhibitor. These *in vitro* data relative to the value of MFI and the responsiveness of leukaemic cells to chemotherapeutics find correspondence in studies carried out *in vivo* where the development of chemoresistance was observed in patients who *a priori* had shown a value of greater than 25 and MFI for which the clinical course confirmed the development of chemoresistance.

In particular, the studies carried out by the authors have made it possible to define an MFI value that is predictive of the development of chemoresistance or useful for the monitoring of chemoresistance itself, before, after or during a particular treatment protocol. Specifically, a MFI index greater than or equal to 25 is a predictive index of the development of chemoresistance and, in the case of monitoring during treatment, the persistence of chemoresistance. The MFI value higher than 25 will be hence an idnex of a more effective treatment based on the compositions defined and described herein. The following examples and experimental results are intended to indicate the modes of carrying out the present description without being limitative of the same.

### EXAMPLES AND EXPERIMENTAL RESULTS

Only the combination of erythromycin with doxorubicin or a cortisone-based compound selected from prednisone, prednisolone, dexamethasone, betamethasone or cortisol falls within the scope of the claims.

Despite improvements in treatment options, resistance to chemotherapy remains one of the biggest obstacles to effective treatment in a significant proportion of children with acute lymphoblastic leukaemia (ALL), particularly in those with recurrent ALL. The bone marrow mesenchymal cells (MSC) can contribute to create drug resistance in leukaemia cells and various mechanisms have been proposed to explain this effect such as the molecular interaction between the factor derived from the stroma 1α (SDF-1α) and its receptor CXCR4 that may trigger the involvement of integrin and the activation of downstream signaling cascades that promote the survival of leukaemia cells. There are recent evidences indicating that integrins can form macromolecular complexes with ion channels, and that the resulting channel/integrin complex may regulate cell survival. Among the ion channels, those encoded by the *ether-a-go-go-related gene* 1, hERG1 channels, have been shown to form protein complexes with integrins in many types of cancer cells. In experiments with ALL cell lines REH, RS4, 11 and 697 the authors found that exposure of ALL cells with MSC induced the expression of a signalling complex on the plasma membrane formed by hERG1 channels, □β1 integrin subunit and the chemokine receptor CXCR4 on the surface of ALL cells. This protein complex triggered activation of intracellular pro-survival pathways. The authors found that the hERG1 channels are central to this protection mechanism. The three cell lines and all cases of primary ALL (n = 63) expressed hERG1, and exposure to blockers of hERG1 could abrogate this protective effect of MSC and strongly increase the cytotoxicity of chemotherapeutic drugs commonly used to treat ALL such as doxorubicin, prednisone and methotrexate. In fact, MSC mediated chemoresistance may be overcome by several hERG1 blockers including classic class III antiarrhythmics, such as E4031 and Way 123.398 , as well as other agents classified as blocking agents of hERG1 such as sertindole and erythromycin. These findings were observed in both ALL cell lines, and in primary ALL cells and have been confirmed by studies in mouse models of ALL. In particular, blockers of hERG1 could overcome drug resistance mediated by MSC, in ALL cells injected into immunodeficient mice: mice treated with hERG1 blockers showed a marked increase in the rate of apoptosis of ALL cells in the bone marrow, reduced weight and reduced leukaemic infiltration of ALL cells in the liver and bone marrow. In addition, hERG1 blockers also improved the anti-leukaemic effect of corticosteroids in mice injected with cells resistant to corticosteroids (the cells of the cell line REH). In fact, E4031 reduced engraftment in the bone marrow, and this effect was related to an increase of apoptosis of ALL cells and was higher than that produced by dexamethasone alone.

Treatment with E4031 and dexamethasone almost abolished the development of leukaemia in mice. In short, the block of hERG1 results in preventing the growth of ALL cells and increases the effect of chemotherapy against ALL. Since some hERG1 inhibitors that have shown to be effective are available for medical use and no risk of serious cardiac arrhythmia should be considered for the treatment of ALL drug resistance. The basis of this invention is the identification of the presence of hERG1 channel on the plasma membrane of leukaemic cells, and its ability to form macromolecular complexes with other membrane proteins, in particular with integrins containing the subunit beta1 (like as VLA4 and VLA5), growth factor receptors and chemokine receptors (see publications 7 and 5).

The signalling activity of these complexes, and, within these, the functionality of the hERG1 channel, has been proved to be critical to regulate the survival of leukaemia cells in the bone marrow. We recall once again that the normal and leukaemic hematopoietic cells reside in the bone marrow in specialized areas, called "niches" that provide the structural and physiological conditions for their growth and survival.

The adhesion mechanisms that facilitate the survival of the stem cells and of the leukaemic cells in the bone marrow, and conversely their migration through the stromal layers and then out of the bone marrow, including the integrin VLA4. This process is also directed by SDF1α chemokine secreted by bone marrow stroma and by its receptor CXCR4, present on the membrane of leukaemic cells. The experiments conducted by the inventors have been performed on human leukaemia cells: REH, RS and 697.

In all the cell lines analyzed, they found, by co-immunoprecipitation experiments, the formation of a multiprotein complex formed by the chemokine receptor CXCR4, the integrin beta 1 subunit and the hERG1 channel.

CXCR4/hERG1 complex formation was stimulated by ligand (SDF1α) binding to its receptor CXCR4; on the other hand, the β1 integrin subunit is associated with the CXCR4/hERG1 complex only after integrin activation. In addition, the co-culture of the cell lines of type B lymphoblastic leukaemia with bone marrow stromal cells produces a significant increase of the association of the integrin to the complex CXCR4/hERG1.

The inventors also demonstrated that the protein ILK (integrin-linked kinase) is expressed in the cell lines of type B acute lymphoblastic leukaemia (ALL-B), cultured under standard culture conditions, and that the activity of this kinase is increased by both stimulation with SDF1α and integrin activation, as well as by co-culture with human bone marrow stromal cells, and that this activation is mediated by integrins.

It was also shown that B-ALL leukaemia cells activate the phosphorylation of MAPK and Akt when cultured with marrow stromal cells and that this activation was integrin-dependent; the addition of hERG1 inhibitors significantly inhibited the activation induced by the stroma of these signaling pathways.

### Example 1

### Co-culture of stromal cells and leukaemic cells

Cells used in the experiments have been obtained and amplified from aliquots of a stromal cell line, obtained from St. Jude Children's Hospital di Memphis (USA). Cells were resuspended at 2 X 10⁶/ml in RPMI-1640 that contained 10% fetal bovine serum (FCS, *Hyclone),* 2 mmol/l of L-glutamine (*Euroclone),* 1% of penicillin-streptomycin (*Euroclone*) and 10⁻⁶ mol/l of hydrocortisone (*Sigma,* St Louis, MO, USA). Stromal cells were incubated at 37° C, 5% CO₂ and 90% humidity and after the formation of confluent layers, about after one week of culture, cells were detached and used for experiments of co-culture with leukaemia cell lines in 96-well flat-bottom plates.

The day before the experiment, the wells were coated with 0,1 % fibronectin (*Sigma*) at the final concentration of 1 µg/well to allow the adhesion of the cells. 10 µl of fibronectin (diluted in PBS) have been added in each well and plates left open overnight, in a laminar flow hood, to air dry.

The day after, the media was removed from the MSC and adherent cells were washed with PBS and incubated for two minutes in the incubator, to allow a partial detachment.

Then PBS was removed and 5 ml trypsin (*Euroclone*) were added and further incubated at 37°; after one minute, the flask was vigorously shaken, until complete cell detachment. Then, cells were washed once with RPMI-1640 with 10%FBS, 2 mmol/l of L-glutamine (*Euroclone*), 1% of penicillin-streptomycin (*Euroclone*) and resuspended in fresh stroma culture medium containing 10⁻⁶ mol/l of hydrocortisone (*Sigma*) and seeded in 96-well flat-bottom plates 200 µl/well.

The day after, leukaemic cell counting was performed, taking 20 µl of cell suspension from the flask and adding 20 µl of Trypan Blue (ratio 1:1). The total number of cells and percent viability was determined using the Burker chamber.

After removing the 50% of culture medium, and adding an equal volume of fresh medium without hydrocortisone (step repeated for seven times), cell suspension was plated at 100.000 cells/well, after centrifuging at 1200 rpm for 5 minutes and resuspension in AIM-V medium (*Gibco*). For each cell line, both wells containing stromal cells and leukaemic cells and wells containing only leukaemic cells were prepared.

### Example 2

### Test of the pharmacological effects on cell co-cultures obtained in the example 1.

To test *in vitro* effects, the following drugs were added to the wells for each cell line obtained in example 1: hERG1 inhibitors (E4031 20 µM, Way 20 µM, erythromycin, 100 µM, sertindole (1µM)), doxorubicin (0.1 µg/ml) (Amersham, GE Healthcare); prednisone (5 µM) (Sigma-Aldrich); methotrexate (1,5 µM).

For cell counting, after 48 hours of incubation at 37° C and 5% di CO₂, cells were recovered from the plates. We next evaluated the apoptosis. During apoptosis, a series of changes of plasma membrane occurs, one of these alterations of plasma membrane is the shift of phosphatidylserine (PS) from the inner to the outer surface of the cell. The recognition of PS by macrophage allows them to engulf the apoptotic cell, preventing the inflammatory process. The analysis of PS on the plasma membrane of apoptotic cells is performed by using annexin V-fluorescein and propidium iodide (PI) assay (Annexin-V-Fluos Staining Kit, *Roche*).

Annexin is a Ca²⁺-dependent phospholipid-binding protein with high affinity for phosphatidylserine, such characteristic permits to differentiate apoptotic cells from viable cells. Since necrotic cells also expose PS according to the loss of membrane integrity, it is necessary another marker to differentiate the two cellular populations.

To this purpose, propidium iodide is used, since it stains only necrotic cells, allowing to discriminate between apoptotic and necrotic cells.

The protocol of annexin requie, first, the preparation of the reaction mix, containing 20 µl of annexin and 20 µl of propidium iodide diluted in 1 ml of incubation buffer (1 ml of such mix is enough for 10 samples). About 10⁶ cells are washed with PBS and centrifuged at 200g for 5 minutes; the cell pellet is then resuspend in 100 µl of the solution previously described and incubated for 10-15 min at 15-25°C. The samples are resuspended in 500 µl of incubation buffer, before proceeding to flow cytometry analysis.

### Evaluation of apoptosis

Milestones to the present invention have been experiments in which the role of the hERG1 channel on survival of leukaemic cells has been determined, by assessing apoptosis.

During apoptosis, a series of changes on the plasma membrane occurs, one of the earliest alterations of the plasma membrane (defined as early apoptosis) is the shift of phosphatidylserine (PS) from the inner, to the outer surface of the cell. This assay evaluates PS on the plasma membrane of apoptotic cells by using annexin V-fluorescein (that binds PS, see Materials and Methods) and propidium iodide.

Since necrotic cells also expose PS as a consequence of the loss of membrane integrity, another marker is necessary in order to differentiate the two cellular populations and then propidium iodide is used. Propidium iodide stains necrotic cells only and hence allows to discriminate between apoptotic and necrotic cells.

We then identified three cell populations: i) viable cells (annexin V-/propidium iodide-), ii) apoptotic cells (early apoptosis) (annexin V +), and iii) cells in late apoptosis or necrotic (annexin V + / propidium iodide+). All the cell lines (697, RS, REH) were cultured in the absence and in the presence of human bone marrow stromal cells (MSC) in order to reproduce as closely as possible the condition that occurs in the bone marrow.

Experiments were also performed adding drugs commonly employed to treat leukaemia (doxorubicin (0.1 µg/ml); prednisone (5 µM); methotrexate (1,5 mM)), in the absence or in the presence of hERG1 blockers (E4031, 20 µM; Way 123,398, 20 µM; erythromycin, 100 µM; sertindole (1µM)). After 48 hours of incubation, cells were separated from the stroma and processed for the analysis of apotosis.

### Example 3

### Effects of the combinations of antitumoral drugs and hERG1 inhibitors

In Figure 1 are shown the main results for the purposes of the present invention. In panel A of the figure, it is possible to notice that the proapototic effect of the drug doxorubicin (DOXO) on 697 cells (first white bar on the left) is almost completely abolished when leukaemic cells are cultured in the presence of bone marrow stromal cells (MSC) (grey bar). Such protective effect exerted by the stroma is reverted when the co-culture with stromal cells is performed in the presence of the specific hERG1 inhibitor, E4031 (black bar on the left), with a significant level of p < 0.001. A similar MSC- induced protective effect is exerted, although at different levels, by the drugs prednisone (PDN) and methotrexate (MTX). It must be noticed that E4031, alone, has a pro-apoptotic effect (last bar, in grey, in panel A), that is strongly strengthened by the combination with doxorubicin. In panel B are reported similar data, obtained in 697 cells treated with doxorubicin, in the absence and in the presence of MSC, in the absence and in the presence of other two hERG1 inhibitors: erithromycin and sertindole. It must be noticed that such drugs, although blocking hERG1 channels, have no effect of lengthening the QT, and hence have no of those cardiotoxic effects, that, on the contrary, class III anthiarrhythmics, like E4031, can have.

In panel C the effect of the combination doxorubicina+ E4031 (or + erythromycin) on leukaemia cells ALL (ALL(3), ALL(4), ALL(6) is reported. Also in these cases, the pro-apoptotic effect of the pharmacological combination is clearly evident, and overcomes MSC-induced chemoresistence (see black bars in all panels of Figure 1 C). Such protective effect on MSC- induced chemoresistance is less evident in ALL(6) cells.

In panel C of Figure 1 are also shown the MFI values relative to the primary samples analysed, determined as described in the example 4. It is possible to stress the correspondence between a greater effect of the combination anticancer drug+hERG1 inhibitor, and an higher MFI index.

### Example 4

### Evaluation and determination of MFI

Effects of hERG1 blockers on apoptosis induced by doxorubicin in human leukaemic cells cultured *in vitro* in the absence or in the presence of marrow stromal cells (MSC), and treated (indicated with +) or not (indicated with -) with E4031, Erithromycin, Sertindole and Way. A, B) 697 cell line; C) ALL(3), ALL(4) and ALL(6) cells. Aliquots of 1x10⁵ cells were centrifuged at 1100 rpm for 5 minutes, washed with PBS 1X and then labelled at room temperature for 15 minutes with the primary antibody (mouse monoclonal, anti hERG1, produced in the inventor's laboratory, specific for an extracellular epitope of the channel, diluted 1:50). At the end of the incubation, cells were washed with PBS 1X and then stained for 15 minutes with the secondary antibody (anti-mouse IgG FITC (1 µg/10⁶cells)). The pellet was washed twice with PBS 1X and resuspended in 500 µl of a solution of 1% formalin in PBS. The analysis was performed with the flow cytometer FACScan (*Becton Dickinson*). The inventors standardized a new method for the detection and absolute quantification of the hERG1 channel on the plasma membrane, regardless of the flow cytometer used, of the voltage of photomultiplier and of the user. The detection of the antigen investigated is obtained by using fluorescent tracers that generate a signal, which is translated in terms of intensity of fluorescence.

The Mean Fluorescence Index (MFI) is defined as the ratio between the mean fluorescence of the sample under analysis and the fluorescence of a sample incubated only with the secondary antibody (that is fluorescent and hence the sample shows a signal which is not due to the direct antigen-antibody binding). In other words, such index can be calculated as the ratio between the specific signal of the sample which has been labelled with both the primary and secondary antibody (mean fluorescence of the sample = FC) and the aspecific signal obtained in the same sample once labelled only with the secondary antibody (mean fluorescence of the control = Fc), multiplied by 100: hence MFI = FC/Fc 100. The values hence obtained, expressed in a percentage scale, give an estimate which is independent of the fluorophore linked to the secondary antibody (in fact one can use Cy3, Cy5, Alexa 488, PE, other than FITC) and of the flow cytometer which has been used.

From results obtained from experiments performed on cells from patients whose clinical course is known, it emerged that a MFI value higher or equal to 25 is index of the presence of future development of chemoresistance.

### EXAMPLE 5

### In Vivo verification of the co-culture method

In Figure 2 the effects of corticosteroids, hERG1 inhibitors and combination of corticosteroids/hERG1 inhibitors in an *in vivo* model of human leukaemic disease (human leukaemic cells REH inoculated in NOD / SCID mice) is shown. The cells were inoculated by injection into the tail vein and seven days after injection, the animals were treated with E4031, dexamethasone, dexamethasone + E4031 for two weeks; the control mice were treated, according to the same protocol, only with saline.

At the end of two weeks of treatment the animals were sacrificed and the spleens and bone marrows were removed. The bone marrow engraftment was assessed by measuring the percentage of hCD45 + cells versus mCD45 + cells by flow cytometry. The Tunel assay for evaluation of apoptosis was performer on bone marrow histological sections from the control and treated animals.

### REFERENCES

1 Konopleva, M et al. Stromal cells prevent apoptosis of AML cells by up-regulation of anti-apoptotic proteins. Leukaemia 16: 1713-1724 (2002);
2 Zeng, Z et al. Inhibition of CXCR4 with the novel RCP168 peptide overcomes stroma-mediated chemoresistance in chronic and acute leukaemias. Mol. Cancer Ther. 5: 3113-3121 (2006);
3 Tabe, Y et al. Activation of integrin-linked kinase is a critical prosurvival pathway induced in leukaemic cells by bone marrow-derived stromal cells. Cancer Res. 67: 684-694 (2007);
4 Arcangeli, A Becchetti, A. Complex functional interaction between integrin receptors and ion channels. Trends Cel.l Biol. 16: 631-639 (2006);
5 Arcangeli, A et al. Targeting ion channels in cancer: a novel frontier in antineoplastic therapy. Cur. Med. Chem. 16(1): 66-93 (2009).
6 Pillozzi S., et al. HERG potassium channels are constitutively expressed in primary human acute myeloid leukaemias and regulate cell proliferation of normal and leukaemic haemopoietic progenitors. Leukaemia, 16, 1791-1798, (2002);
7 Pillozzi S, et al. VEGFR-1 (FLT-1), b1 integrin and hERG K+ channel form a macromolecular signaling complex in acute myeloid leukaemia: role in cell migration and clinical outcome. Blood, 110: 1238-1250, (2007).
8 Abrams Ra, et al. Ficoll-Hypaque separation of bone marrow cells. Blood, 1985 66: 472-473.
9 Mariabe A. et al. Bone marrow-derived stromal cells prevent apoptotic cell death in B-lineage acute lymphoblastic leukaemia. Blood, 1992 May 1;79(9):2370-7.

## Claims

1. A mixture comprising a hERG1 channels blocker in combination with one or more compounds for anticancer therapy, for use in the treatment of chemoresistant and/or potentially chemoresistant leukaemias wherein such hERG1 channels blocker is erythromycin and such compound for anticancer therapy is selected in the group: doxorubicin, cortisone-based compounds
wherein said cortisone-based compound is selected in the group: prednisone, prendisolone, dexamethasone, betamethasone, cortisol.

2. The mixture for use according to claim 1 wherein said hERG1 channels blocker is erythromycin and said compound for anticancer therapy is doxorubicin.

3. The mixture for use according to any one of claims 1 or 2 wherein said chemoresistant and/or potentially chemoresistant leukaemias are selected in the group: paediatric and adult leukaemias such as paediatric or adult acute lymphoblastic leukaemia, paediatric and adult acute myeloid leukaemia, adult chronic lymphoblastic leukaemia and adult chronic myeloid leukaemia.

4. A pharmaceutical composition comprising the mixture according to any one of claims 1 to 3 for use in the treatment of chemoresistant and/or potentially chemoresistant leukaemias.

5. The composition for use according to claim 4 further comprising one or more pharmaceutically acceptable carrier and/or diluent and/or excipient.

6. The composition for use according to any one of claims 4 or 5 for parenteral, oral, nasal, aerosol, systemic administration.

7. The composition for use according to any one of claims 4-6 wherein said chemoresistant and/or potentially chemoresistant leukaemias are selected in the group: adult and paediatric leukaemias such as paediatric or adult acute lymphoblastic leukaemia, paediatric and adult acute myeloid leukaemia, adult chronic lymphoblastic leukaemia and adult chronic myeloid leukaemia.

8. The composition for use according to any one of claims 4-7 in a form selected in the group of suspension, emulsion, cream, spray, granules, powder, solution, capsule, tablet, tablet, lyophilized, pill.

9. The composition for use according to any one of claims 4-8 wherein the amount of said hERG1 channels blocker in said composition per unit dose is such that it is administered in an amount between 0.3 and 100 mg/kg/day.

10. The composition for use according to any one of claims 4-9 wherein the amount of said anti-cancer compound in said composition is between 100% and 50% of the amount used as a typical dosage of this compound in cancer therapy.

## Patentansprüche

1. Gemisch, das umfasst: einen hERG1-Kanal-Blocker in Kombination mit einer oder mehreren Verbindungen zur Krebstherapie zur Verwendung bei der Behandlung von chemoresistenten und/oder potentiell chemoresistenten Leukämien, wobei ein solcher hERG1-Kanal-Blocker Erythromycin ist und eine solche Verbindung zur Krebstherapie aus der folgenden Gruppe gewählt wird: Doxorubicin, cortisonbasierte Verbindungen,
wobei die cortisonbasierte Verbindung aus der folgenden Gruppe gewählt wird: Prednison, Prednisolon, Dexamethason, Betamethason, Cortisol.

2. Gemisch zur Verwendung gemäß Anspruch 1, wobei der hERG1-Kanal-Blocker Erythromycin und die Verbindung zur Krebstherapie Doxorubicin ist.

3. Gemisch zur Verwendung gemäß einem der Ansprüche 1 oder 2, wobei die chemoresistenten und/oder potentiell chemoresistenten Leukämien aus der folgenden Gruppe gewählt werden: pädiatrische und adulte Leukämien, wie zum Beispiel pädiatrische oder adulte akute lymphoblastische Leukämie, pädiatrische und adulte akute myeloische Leukämie, adulte chronische lymphoblastische Leukämie und adulte chronische myeloische Leukämie.

4. Pharmazeutische Verbindung, die ein Gemisch gemäß einem der Ansprüche 1 bis 3 zur Behandlung von chemoresistenten und/oder potentiell chemoresistenten Leukämien umfasst.

5. Verbindung zur Verwendung gemäß Anspruch 4, die weiterhin einen oder mehrere pharmazeutisch unbedenkliche Träger und/oder Verdünnungsmittel und/oder Hilfsstoffe umfasst.

6. Verbindung zur Verwendung gemäß einem der Ansprüche 4 oder 5 zur parenteralen, oralen, nasalen, Ärosol- und systemischen Verabreichung.

7. Verbindung zur Verwendung gemäß einem der Ansprüche 4 - 6, wobei die chemoresistenten und/oder potentiell chemoresistenten Leukämien aus der folgenden Gruppe gewählt werden: adulte und pädiatrische Leukämien, wie zum Beispiel pädiatrische oder adulte akute lymphoblastische Leukämie, pädiatrische und adulte akute myeloische Leukämie, adulte chronische lymphoblastische Leukämie und adulte chronische myeloische Leukämie.

8. Verbindung zur Verwendung gemäß einem der Ansprüche 4 - 7 in einer Form, die aus der folgenden Gruppe gewählt wird: Suspension, Emulsion, Creme, Spray, Granulat, Puder, Lösung, Kapsel, Tablette, Lyophilisat, Pille.

9. Verbindung zur Verwendung gemäß einem der Ansprüche 4 - 8, wobei die Menge des hERG1-Kanal-Blockers in der Verbindung pro Einzeldosis so ist, dass er in einer Menge von zwischen 0,3 und 100 mg/kg/Tag verabreicht wird.

10. Verbindung zur Verwendung gemäß einem der Ansprüche 4 - 9, wobei die Menge der Verbindung zur Krebstherapie in der Zusammensetzung zwischen 100 % und 50 % von der Menge beträgt, die als eine typische Dosierung dieser Verbindung bei der Krebstherapie verwendet wird.

## Revendications

1. Mélange comprenant un bloqueur des canaux de hERG1 en combinaison avec un ou plusieurs composés pour un traitement anticancéreux, pour une utilisation dans le traitement de leucémies chimiorésistantes et/ou potentiellement chimiorésistantes dans lequel un tel bloqueur des canaux de hERG1 est l'érythromycine et un tel composé pour un traitement anticancéreux est choisi dans le groupe : doxorubicine, composés à base de cortisone
où ledit composé à base de cortisone est choisi dans le groupe : prednisone, prednisolone, dexaméthasone, bétaméthasone, cortisol.

2. Mélange pour une utilisation selon la revendication 1, dans lequel le bloqueur des canaux de hERG1 est l'érythromycine et ledit composé pour un traitement anticancéreux est la doxorubicine.

3. Mélange pour une utilisation selon l'une quelconque des revendications 1 ou 2, où lesdites leucémies chimiorésistantes et/ou potentiellement chimiorésistantes sont choisies dans le groupe : leucémies pédiatriques et de l'adulte comme la leucémie lymphoblastique aiguë pédiatrique ou de l'adulte, la leucémie myéloïde aiguë pédiatrique et de l'adulte, la leucémie lymphoblastique chronique de l'adulte et la leucémie myéloïde chronique de l'adulte.

4. Composition pharmaceutique comprenant le mélange selon l'une quelconque des revendications 1 à 3 pour une utilisation dans le traitement de leucémies chimiorésistantes et/ou potentiellement chimiorésistantes.

5. Composition pour une utilisation selon la revendication 4, comprenant en outre un ou plusieurs supports et/ou diluants et/ou excipients pharmaceutiquement acceptables.

6. Composition pour une utilisation selon l'une quelconque des revendications 4 ou 5, pour une administration parentérale, orale, nasale, par aérosol, systémique.

7. Composition pour une utilisation selon l'une quelconque des revendications 4 à 6, où lesdites leucémies chimiorésistantes et/ou potentiellement chimiorésistantes sont choisies dans le groupe : leucémies de l'adulte et pédiatriques comme la leucémie lymphoblastique aiguë pédiatrique ou de l'adulte, la leucémie myéloïde aiguë pédiatrique et de l'adulte, la leucémie lymphoblastique chronique de l'adulte et la leucémie myéloïde chronique de l'adulte.

8. Composition pour une utilisation selon l'une quelconque des revendications 4 à 7, sous une forme choisie dans le groupe de suspension, émulsion, crème, pulvérisation, granulés, poudre, solution, capsule, comprimé, lyophilisée, pilule.

9. Composition pour une utilisation selon l'une quelconque des revendications 4 à 8, dans laquelle la quantité dudit bloqueur des canaux de hERG1 dans ladite composition par dose unitaire est telle qu'il est administré dans une quantité située entre 0,3 et 100 mg/kg/jour.

10. Composition pour une utilisation selon l'une quelconque des revendications 4 à 9, dans laquelle la quantité dudit composé anticancéreux dans ladite composition se situe entre 100 % et 50 % de la quantité utilisée comme dosage type de ce composé dans un traitement anticancéreux.
